# EUROPEAN PATENT APPLICATION

(11) **EP 1 905 782 A1**
(43) Date of publication of application: **02.04.2008**
(21) Application number: 07012625.5
(22) Date of filing: 18.03.1992
(51) Int. Cl.: C07K 16/24, A61K 39/395, C12N 15/62, C12N 15/13, C12N 5/10, C12N 1/21, C07K 16/46

(54) **Monoclonal and chimeric antibodies specific for human tumor necrosis factor**

(30) Priority: 18.03.1991 US 670827
(62) Divisional of application: 00204461.8
(71) Applicant: NEW YORK UNIVERSITY, New York, NY 10016 (US); CENTOCOR, INC., Malvern, PA 19355-1307 (US)
(72) Inventor: Vilcek, Jan, New York, NY 10021 (US); Le, Junming, New York, NY 11375 (US); Daddona, Peter, Menlo Park, CA 94025 (US); Siegel, Scott, Ringoes, NJ 08551 (US); Knight, David, Berwyn, PA 19312 (US); Ghrayeb, John, Downington, PA 19335 (US)
(74) Representative: Schönbohm, Carla

(57) **Abstract**

Anti-TNF antibodies, fragments and regions thereof which are specific for human tumor necrosis factor-α (TNFα) and are useful *in vivo* diagnosis and therapy of a number of TNPα-mediated pathologies and conditions, as well as polynucleotides coding for murine and chimeric antibodies, methods of producing the antibody, methods of use of the anti-TNF antibody, or fragment, region or derivative thereof, in immunoassays and immunotherapeutic approaches are provided.

## Description

### BACKGROUND OF THE INVENTION

This application is a continuation of U.S. application serial No. 07/670,827, filed March, 18, 1991, which is entirely incorporated by reference.

### FIELD OF THE INVENTION

The present invention in the field of immunology and medicine relates to antibodies which are specific for human tumor necrosis factor-alpha (hTNFα) ; fragments, regions and derivatives thereof; and to pharmaceutical and diagnostic compositions and production, diagnostic and therapeutic methods thereof. The invention further relates to nucleotide sequences encoding such antibodies, fragments and regions, as well as vectors and hosts containing such sequences, and methods thereof .

### DESCRIPTION OF THE BACKGROUND ART

The cytokine known as tumor necrosis factor-α (TNFα; also termed cachectin) is a protein secreted primarily by monocytes and macrophages in response to endotoxin or other stimuli as a soluble homotrimer of 17 kD protein subunits (smith, R.A. at al., J. Biol. Chem. 262 :6951-6954 (1987)). A membrane-bound 26 kD precursor form of TNF has also been described (Kriegler, M. et al., Cell 53:45-53 (1988)). For reviews of TNF, see Beutler, B. et al., Nature 320:564 (1986), Old, L.J., Science 230:630 (1986), and Le, J. et al., Lab. Invest. 56:234 (1987). TNF was originally discovered in the serum of animals injected sequentially with a bacterial vaccine (bacillus Calmette- Guerin, BCG) and endotoxin (Carswell, E.A. et al., Proc. Natl. Acad. Sci. USA 72:3666 (1975)).

The expression of the gene encoding TNFα is not limited to cells of the monocyte/macrophage family. several human non-monocytic tumor cell lines were shown to produce TNF (Rubin, B.Y. et al., J. Exp. Med. 164:1350 (1986); Sprigs, D. et. al., Proc. Natl. Acad. Sci. USA 84:6563 (1987)). TNF is also produced by CD4⁺ and CD8⁺ peripheral blood T lymphocytes, and by various cultured T and B cell lines (Cuturi, M.C-, et al., J. Exp. Med. 165:1581 (1989); Sung, S.-S.J. et al., J. Exp. Med. 168:1539 (1988)).

Accumulating evidence indicates that TNF is a regulatory cytokine with pleiotropic biological activities. These activities include: inhibition of lipoprotein lipase synthesis ("cachectin" activity) (Beutler, B. et al., Nature 316:552 (1985)), activation of polymorphonuclear leukocytes (Klebanoff, S.J. et al., J. Immunol. 136:4220 (1986); Perussia, B., et al., J. Immunol. 138:765 (1987)), inhibition of cell growth or stimulation of cell growth (Vilcek, J. et al, J. Exp. Med. 163:632 (1986); Sugarman, B.J. et al. Science 230:943 (1985); Lachman, L.B. et al., J. Immunol, 138:2913 (1987)), cytotoxic action on certain transformed cell types (Lachman, L.B. **et al.,** supra; Darzynkiewicz, Z. et al., Canc. Res. 44:83 (1984)), antiviral activity (Kohase, M. et al., Cell 45:659 (1986); Wong, G.H.W. et al. , Nature 323 : 819. (1986)), stimulation of bone resorption (Bertolini, D.R. et al., Nature 319:516 (1986); 1 Saklatvala, J., Nature 322: 547 (1986)), stimulation of collagenase and prostaglandin E2 production (Dayer, J.-M. et al, J. Exp. Med. 162:2163 (1985)); and immunoregulatory actions, including activation of T cells (Yokota, S. et al., J. Immunol. 140:531 (1988)), B cells (Kehrl, J .H. et. al., J. Exp. Med. 166:786 (1987)), monocytes (Philip, R. et al., Nature 323:86 (1986)), thymocytes (Ranges, G.E. et al., J. Exp. Med. 167:1472 (1988)), and stimulation of the cell-surface expression of major histocompatibility complex (MHC) class I and class II molecules (Collins, T. et al., Proc. Nat1. Acad. Sci. USA 83:446 (1986); Pujol-Borrell, R. et al., Nature 326:304 (1987)).

TNP is noted for its pro-inflamatory actions which result in tissue injury, such as induction of procoagulant activity on vascular endothelial cells (Pober, J.S. et al., J. Immunol. 136 : 1680 (1986)), increased adherence of neutraphils and lymphocytes (Pober, J.S. et. al., J., Immunol. 138:3319 (1987)), and stimulation of the release of platelet activating factor from macrophages, neutrophils and vascular endothelial cells (Camussi, G. et al., J. Exp. Med, 166:1390 (1987)).

Recent evidence implicates TNF in the pathogenesis of many infections (Cerami, A. et al., Immunol. Today 9:28 (1988)), immune disorders, neoplastic pathology, e.g., in cachexia accompanying some malignancies (Oliff, A. et al., Cell 50:555 (1987)), and in autoimmune pathologies and graft-versus host pathology (Piguet, P.-F- et al., J. Exp. Med. 166:1280 (1987)). The association of TNF with cancer and infectious pathologies is often related to the host's catabolic state. A major problem in cancer patients is weight loss, usually associated with anorexia. The extensive wasting which results is known as "cachexia" (Kern, K.A. et al. (J. Parent. Enter. Nutr. 12:286-298 (1988)). Cachexia includes progressive weight loss, anorexia, and persistent erosion of body mass in response to a malignant growth. The fundamental physiological derangement may be related to a decline in food intake relative to energy expenditure. The cachectic state is thus associated with significant morbidity and is responsible for the majority of cancer mortality. A number of studies have suggested that TNF is an important mediator of the cachexia in cancer, infectious pathology, and in other catabolic states.

TNF is thought to play a central role in the pathophysiological consequences of Gram-negative sepsis and endotoxic shock (Michie, H.R. et al., Br. J. Surg. 76:670-671 (1989); Debets, J.M.H. et al., Second Vienna Shock Forum, p.463-466 (1989) ; Simpson, S.Q. et al., Crit. Care Clin. 5 : 27-47 (1989)), including fever, malaise, anorexia, and cachexia. Endotoxin is a potent monocyte/macrophage activator which stimulates production and secretion of TNF (Kornbluth, S.K. et al., J. Immunol., 137:2585-2591 (1986)) and other cytokines. Because TNF could mimic many biological effects of endotoxin, it was concluded to be a central mediator responsible for the clinical manifestations of endotoxin-related illness. TNF and other monocyte-derived cytokines mediate the metabolic and neurohormonal responses to endotoxin (Michie, H.R. et, al., N. Eng, J. Med. 318:1481-1486 (1988)). Endotoxin administration to human volunteers produces acute illness with flu-like symptoms including fever, tachycardia, increased metabolic rate and stress hormone release (Revhaug, A. et al., Arch._Surg. 123:162-170 (1988)). Elevated levels of circulating TNF have also been found in patients suffering from Gram-negative sepsis (Waage, A. et al., Lancet 1:355-357 (1987); Hammerle, A.F. et al., Second Vienna Shock Forum p. 715-718 (1989); Debets, J.M.H. et al., Crit. Care Med. 17:489-497 (1989) ; Calandra, T. et al., J. Infec. Dis. 161:982-987 (1990)). Treatment of cancer patients with TNF (because of its tumoricidal action) revealed that doses greater than 545 µg/m²/24hr caused alterations similar to those induced by injection of endotoxin (4 ng/kg) into healthy humans (Michie, E. R. et al., Surgery 104:280-286 (1988)), supporting TNF's role as the principal host mediator of septic and endotoxemic responses. Chronic intravenous TNF infusion into humans or rats was associated with anorexia, fluid retention, acute phase responses, and negative nitrogen balance (i.e., classic catabolic effects), leading to the conclusion that TWP may be responsible for many of the changes noted during critical illness (Michie, H.R. et al., Ann, Surg. 209:19-24 (1989)).

Passive immunotherapy directed at neutralizing TNF may have a beneficial effect in Gram-negative sepsis and endotoxemia, based on the increased TNF production and elevated TNF levels in these pathology states, as discussed above.

Antibodies to a "modulator" material which was characterized as cachectin (later found to be identical to TNF) were disclosed by Cerami et al. (EPO Patent Publication 0212489, March 4, 1987). Such antibodies were said to be useful in diagnostic immunoassays and in therapy of shock in bacterial infections. Rubin et al. (EPO patent Publication 0218868, April 22, 1967) disclosed monoclonal antibodies to human TNF, the hybridomas secreting such antibodies, methods of producing such antibodies, and the use of such antibodies in imnunoassay of TNF. Yone et al., (EPO Patent Publication 0288088, October 26, 1988) disclosed anti-TNF antibodies, including mAbs, and their utility in immunoassay diagnosis of pathologies, in particular Kawasaki's pathology and bacterial infection. The body fluids of patients with Kawasaki's pathology (infantile acute febrile mucocutaneous lymph node syndrome; Kawasaki, T., Allergy 16:178 (1967) ; Kawasaki, T., Shonica (Pediatrics) 26:935 (1985)) were said to contain elevated TNF levels which were related to progress of the pathology (Yone et al., supra).

other investigators have described mAbs specific for recombinant human TNF which had neutralizing activity in vitro (Liang, C-M. et al. (Biochem. Biophys. Res. Comm. 137:847-854 (1986); Meager, A. et al., Hybridoma 6:305-311 (1987); Fendly at al., Hybridoma 6:359-369 (1987); Bringman, T.S. et al., Hybridoma 6:489-507 (1987); Hirai, M. et al., J. Immunol. Meth.96:57-62 (1987); Moller, A. et al., (Cytokine 2:162.169 (1990)). Some of these mAbs were used to map epitopes of human TNF and develop enzyme immunoassays (Fendly et al., supra; Hirai et al., supra ; Moller et al., supra) and to assist in the purification of recombinant TNF (Bringman et al., supra). However, these studies do not provide a basis for producing TNF neutralizing antibodies that can be used for in vivo diagnostic or therapeutic uses in humans.

The most direct support for the use of anti-TNF immunotherapy comes from in vivo protection studies in animals other than humans. Neutralizing antisera or mAbs to TNF have been shown in mammals other than man to abrogate adverse physiological changes and prevent death after lethal challenge in experimental endotoxemia and bacteremia. This effect has been demonstrated, e.g., in rodent lethality assays and in primate pathology model systems (Mathison, J.C. et al., J. Clin. Invest. 81:1925-1937 (1988); Beutler, B. et al., Science 229:869-871 (1985); Tracey, K.J. et al., Nature 330:662-664 (1987); Shimamoto, Y. et al., Immunol. Lett. 17:311-318 (1988); Silva, A.T. et al., J. Infect. Dis. 162: 421-427 (1990) ; Opal, S.M. et al., J. Infect. Dis. 161:1148-1152 (1990); Hinshaw, L.B. et al., Circ. Shock 30:279-292 (1990)). For example, F(ab')₂ fragments of neutralizing anti-TNF mAbs were able to prevent septic shock produced by live E. coli in baboons (Tracey, K.J. et al., supra).

Putative receptor binding loci of hTNF has been presented by Eck and Sprang (J. Biol. Chem. 264 (29), 17595-17605 (1989), who identified the receptor binding loci of TNF-α as consisting of amino acids 11-13, 37-42, 49-57 and 155-157. PCT application WO91/02078 (priority date of August 7, 1989) discloses TNF ligands which can bind to monoclonal antibodies having the following epitopes : at least one of 1-20, 56-77, and 108-127; at least two of 1-20, 56-77, 108-127 and 138-149 ; all of 1-18, 58-65, 115-125 and 138-149; all of 1-18, and 108-128; all of 56-79, 110-127 and 135- or 136-155; all of 1-30, 117-128 and 141-153; all of 1-26, 117-128 and 141-153; all of 22-40, 49-96 or -97, 110-127 and 136-153; all of 12-22, 36-45, 96-105 and 132-157; all of both of 1-20 and 76-90; all of 22-40, 69-97, 105-128 and 135-155; all of 22-31 and 146-157; all of 22-40 and 49-98; at least one of 22-40, 49-98 and 69-97, both of 22-40 and 70-87.

To date, experience with anti-TNF mAb therapy in humans has been limited. In a phase I study, fourteen patients with severe septic shock were administered a neutralizing mouse anti-TNF mAb in a single dose from 0.4-10 mg/kg (Exley, A.R. et al., Lancet 335:1275-1277 (1990)) . However, seven of the fourteen patients developed a human anti-murine antibody response to the treatment, which treatment suffers from the known problems due to immunogenicity from the murine heavy and light chain portions of the antibody. Such immunogenicity causes decrease effectiveness of continued administration and can render treatment ineffective due to the increased immune rejection response in patients undergoing diagnostic or therapeutic administration of mouse anti-TNF antibodies.

Administration of murine TNF mAb to patients suffering from severe graft vs. host pathology has also been reported (Herve, P. et al.,Lymphoma Res, 9 : 591 (1990)).

Accordingly, there is a need to provide novel TNF antibodies that overcome the problems of murine antibody immunogenicity and which provide reduced immunogenicity and increased neutralization activity.

### SUMMARY OF THE INVENTION

It is an object of the present invention to overcome the deficiencies of the background art.

It is also an object of the present invention to provide anti-tissue necrosis factor (TNF) murine antibodies and chimeric antibodies, and fragments and regions thereof, which inhibit or neutralize TNF biological activity *in vivo* and are specific for human tumor necrosis factor-alpha (hTNFα). In a preferred embodiment, anti-TNF antibodies of the present invention competitively inhibit the binding of A2 antibodies to TNF.

In another preferred embodiment, anti-TNF antibodies of the present invention bind an epitope of at least 5 amino acids of residues 87-108 or both of residues 59-80 and 87-108 of hTNFα (of SEQ ID NO:1), but which do not bind known or putative receptor binding portions of TNF, such as amino acid sequences 1-20, 11-13, 37-42, 49-57 or 155-157 of TNF (of SEQ ID NO:1).

Such anti-TNF antibodies, fragments and regions thereof of the present invention include those produced both from hybridomas, such as murine-murine hybridomas, and from recombinant cells expressing heterologous nucleic acid encoding at least variable regions, such as murine-human chimeric antibodies, of such TNF specific antibodies, fragments or regions of the present invention.

Is a further object of the present invention to provide anti-TNF antibodies, fragments, and regions using hybridoma and genetic engineering technologies to provide useful products for the in vivo treatment and diagnosis of human pathologies associated with TNF, as described above.

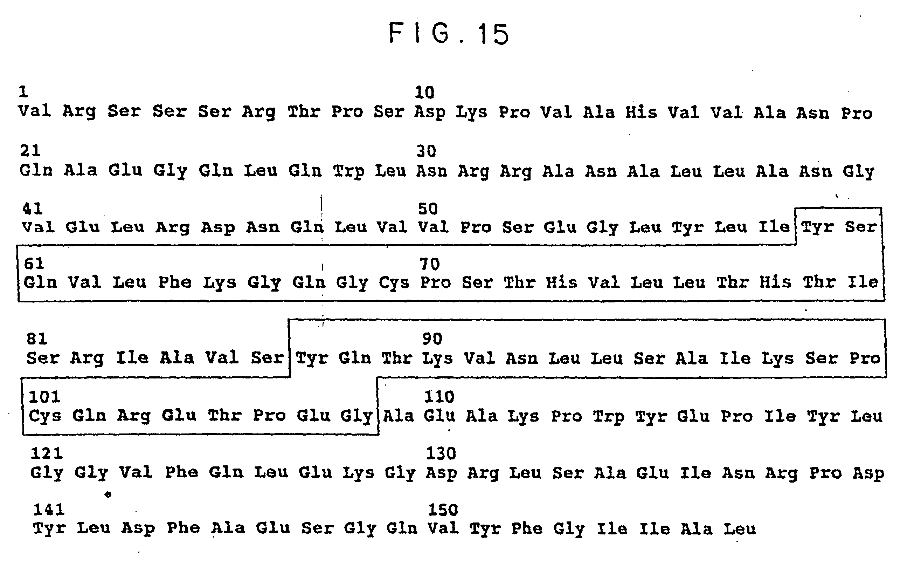

It is a further object of the prevent invention to provide antigenic polypeptides of hTNFα which correspond to portions of TNF-α that, when epitopes of these polypeptides are bound by TNf-specific antibodies, fragments or regions which neutralize or inhibit the biological activity of TNF-α *in* vivo. It is another object of the present invention to provide antibodies generated from hybridomas or recombinant hosts that bind specific epitopes of polypeptides corresponding to at least 5 amino acids selected from residues 87-108 or both residues 59-80 and 87-108 of hTNFα (of SEQ ID NO:1), as shown in Figure 15.

It is another object of the present invention to provide anti-TNFα antibodies, fragments and regions which have TNFα inhibiting and/or neutralizing activity *in vivo,* and can be provided as pharmaceutical and diagnostic compositions for the diagnosis and treatment of TNFα-related pathologies, including cachexia, acute and chronic infectious and parasitic processes, such as bacterial, viral and fungal infections, acute and chronic inflammatory and immune processes, including autoimmune pathology, alcohol-induced hepatitis, neoplastic pathology and the like. Preferred for therapeutic use are high affinity anti-TNFα antibodies, including recombinantly and hybridoma produced monoclonal and chimeric antibodies, according to the present invention, which inhibit or neutralize human TNF-α with an *in vivo* inhibitory dose-50 (ID50) of at least about 1 *µ*g/ml, more preferably at least about 100 ng/ml, most preferably at least about 15 ng/ml.

According to another aspect of the present invention, anti-TNFα mAbs or chimeric mAbs, or TNFα-binding fragments thereof, which are particularly useful in diagnostic methods for detecting human TNFα in patients suspected of suffering from conditions associated with TNFα production, including methods wherein high affinity murine and/or chimeric anti-TNFα antibodies of the present invention are contacted with biological materials from a patient and an antigen-antibody reaction detected. Also included are kits for detecting TNFα in biological fluids comprising high affinity murine and/or chimeric anti-TNFα antibodies or fragments of the present invention, preferably in detectably labeled form.

The chimeric antibodies of the present invention embody a combination of the advantageous characteristics of mAbs. Like mouse mAbs, they can recognize and bind to human TNF; however, unlike mouse mAbs, the "human-specific" properties of the chimeric antibodies lower the likelihood of an immune response to the antibodies, and result in prolonged survival in the circulation through reduced clearance. Moreover, using the methods disclosed in the present invention, the constant region of any desired human immunoglobulin isotype can be combined with the desired antigen combining site.

In one embodiment, the present invention is directed to a high affinity mouse mAb, designated A2, which is specific for human TNFα. The antibody may be in detectably labeled form. In another embodiment, polypeptide portions of hTNFα are provided that, when bound as part of an intact TNFα molecule by antibodies or fragments specific for epitopes included in these peptide portions, inhibit or neutralize TNFα activity in vivo.

It is also an object of the present invention to provide chimeric immunoglobulin chains, either heavy (H) or light (L), having variable or constant regions, with specificity toward one or more epitopes of TNF, preferably an epitope of at least 5 amino acids of residues 87-107, or a combination of both of 59-80 and 87-108, of hTNFα (of SEQ ID N0:1). In a preferred embodiment, the epitopic amino acids do not include amino acids from residues from at least one of 11-13, 37-42, 49-57 or 155-157 of hTNFα (of SEQ ID NO:1). A human/murine chimeric immunoglobulin chain contains a constant (C) region substantially similar to that present in a natural human immunoglobulin, and a variable (V) region, preferably non-human, having high affinity and the desired specificity for a TNF epitope. The invention also provides antibodies, fragments, and regions having chimeric E and L chains associated so that the overall molecule exhibits the desired antigen recognition and binding properties.

specifically, the present invention is also directed to a chimeric antibody comprising two light chains and two heavy chains, each of the chains comprising at least part of a human constant region and at least part of a variable (V) region of non-human origin having specificity to human TNFα, said antibody binding with high affinity to a inhibiting and/or neutralizing epitope of human TNFα. The invention is also includes a fragment or a derivative such an antibody. Preferably, the V region is of non-human origin, most preferably of marine origin. In a preferred embodiment, the V region is derived from, or binds epitopes of the A2 mAb. In another preferred embodiment, a chimeric antibody is provided which binds epitopes of the antibody designated chimeric A2 (cA2), or a chimeric human-mouse anti-TNF mAb that competitively inhibits the binding of cA2 to TNFα.

Preferably, the chimeric antibody inhibits or neutralizes human TNFα *in vivo* with an ID50 of at least about 1 µg/ml, more preferably at least about 100 ng/ml, most preferably at least about 15, 30, 50, or 80 ng/ml.

### BRIEF DESCRIPTION OF THE DRANINGS

Figure 1 is a graph showing dose dependent binding of mouse mAb A2 to human TNFα.
Figure 2 is a graph showing lack of recognition of heat-inactivated human TNFα by mAb A2.
Figure 3 is a graph showing neutralization of in vitro TNF cytotoxicity by murine A2. Control: murine IgG1 anti-lipid A mAb (8A1) with natural human TNF. Average absorbance values for controls were as follows: no TNF added - 1.08; natural TNF, no antibody - 0.290; and recombinant TNF, no antibody - 0.500.
Figure 4 is a graph showing that mAb A2 and chimeric A2 do not inhibit or neutralize human lymphotoxin (TNFβ).
Figure 5 is a graph showing that mAbs murine A2 and chimeric CA2 do not inhibit or neutralize murine TNFα.
Figures 6 and Figure 7 are graphs showing that mAb A2 inhibits or neutralizes TNF produced by chimpanzee monocytes and rhTNFα.
Figure 8 provides schematic diagrams of the plasmids used for expression of the chimeric H (pA2HG1apgpt) and L (pA2HuKapgpt) chains of the chimeric A2 antibody.
Figure 9 is a graph showing results of a cross-blocking epitope BLISA with murine A2 (mA2) and chimeric (cA2) antibody competitors.
Figure 10 is a graph of a Scatchard analysis of ¹²⁵I-labelled mAb A2 (mA2) and chimeric A2 (cA2) binding to recombinant human TNFα immobilized on a microtiter plate. Each Ka value was calculated from the average of two independent determinations.
Figure 11 is a graph shoving neutralization of TNF cytotoxicity by chimeric A2. The control is a chimeric mouse/human IgGl anti-platelet mAb (7E3) reacting with natural human TNF. Average absorbance values for controls were: no TNF added - 1.08; natural TNF, no Ab = 0.290; and recombinant TNF, no Ab = 0.500.
Figure 12 is a graph showing in vitro neutralization of TNF-induced ELAM-1 expression by chimeric A2. The control is a chimeric mouse/human IgG1 anti-CD4 antibody.
Figure 13 is an amino acid sequence of human TNF as SEQ ID N0:1.
Figure 14A is a graphical representation of epitope mapping of chimeric mAb cA2 indicating relative binding of cA2 to human TNF peptide pins.
Figure 14B is a graphical representation of epitope mapping of chimeric mAb cA2 indicating relative binding of cA2 to human TNF peptide pins in the presence of human TNF.
Figure 15 is an amino acid sequence of human TNF showning sequences having portions of epitopes recognized by cA2.
Figure 16A is a representation of a space filling model of a human TNF monomer.
Figure 16B is a representation of a space filling model of two noncontiguous peptide sequences of human TNF recognized by cA2.

### DETAILED DESCRIPTION OF THE PREFERRED EMBODIMENTS

The present invention provides anti-tissue necrosis factor (TNF) murine antibodies and chimeric murine-human antibodies, and fragments and regions thereof, which inhibit or neutralize TNF biological activity *in* vivo and are specific for human tumor necrosis factor-alpha (hTNFα), which can be used for diagnostic and therapeutic purposes in subjects having pathologies or conditions associated with the presence of a substance reactive with anti-TNF antibody, in particular hTNFα, in amounts exceeding those present in a normal healthy subject. Antibodies, and fragments, regions and derivatives thereof, of the present invention preferably contain at least one V region which recognizes an epitope of TNF which has inhibiting and/or neutralizing biological activity *in vivo.*

Unexpectedly, mAbs of the present invention can block the action of TNF-α without binding to the putative receptor binding locus as presented by Eck and Sprang (J. Biol. Chem. 264 (29), 17595-17605 (1989), as amino acids 11-13, 37-42, 49-57 and 155-157 of hTNF**α** (of SEQ ID NO : 1).

Preferred antibodies of the present invention are murine antibodies or high affinity human-murine chimeric anti-TNFα antibodies, and fragments or regions thereof, that have potent inhibiting and/or neutralizing activity in vivo against human TNFα. Such antibodies and chimeric antibodies include those generated by immunization using purified recombinant hTNF**α** (SEQ ID NO:1) or peptide fragments thereof. Such fragments include epitopes of at least 5 amino acids of residues 87-107, or a combination of both of 59-80 and 87-108 of hTNFα (as these corresponding amino acids of SEQ ID N0:1). Additionally, preferred antibodies, fragments and regions of anti-TNF antibodies of the present invention do not recognize amino acids from at least one of amino acids 11-13, 37-42, 49-57 or 155-157 of hTNFα (of SEQ ID NO:1).

Since circulating concentrations of TNF tend to be extremely low, in the range of about 10 pg/ml in non-septic individuals, and reaching about 50 pg/ml in septic patients and above 100 pg/ml in the sepsis syndrome (Hammerle, A.F. et. al., 1989, supra) or may be only be detectable at sites of TNF-mediated pathology, it is preferred to use high affinity and/or potent in vivo TNF-inhibiting and/or neutralizing antibodies, fragments or regions thereof, for both TNF immunoassays and therapy of TNF-mediated pathology. Such antibodies, fragments, or regions, will preferably have an affinity for hTNFα, expressed as Ka, of at least 10⁴ M⁻¹, more preferably, at least 10⁹ M⁻¹, such as 5 X 10⁸ M⁻¹, 8 X 10⁸ M⁻¹, 2 X 10⁹ M⁻¹, 4 X 10⁹ M⁻¹, 6 X 10⁹ M⁻¹, 8 X 10⁹ M⁻¹.

Preferred for human therapeutic use are high affinity murine and chimeric antibodies, and fragments, regions and derivatives having potent *in vivo* TNFα-inhibiting and/or neutralizing activity, according to the present invention, that block TNF-induced IL-6 secretion. Also preferred for human therapeutic uses are such high affinity murine and chimeric anti-TNFα antibodies, and fragments, regions and derivatives thereof, that block TNF-induced procoagulant activity, including blocking of TNF-induced expression of cell adhesion molecules such as ELAM-1 and ICAM-1 and blocking of TNF mitogenic activity, *in vivo*, *in situ,* and *in vitro.*

Preferred anti-TNF mAbs are those which will competitively inhibit *in vivo* the binding to human TNFα of anti-TNFα murine mAb A2, chimeric mAb cA2, or an antibody having substantially the same specific binding characteristics, as well as fragments and regions thereof. Preferred antibodies of the present invention are those that bind epitopes recognized by A2 and cA2, which are included in amino acids 59-80) and/or 87-108 of hTNFα (as these corresponding amino acids of SEQ ID NO:1), such that the epitopes consist of at least 5 amino acids which comprise at least one amino acid from the above portions of human TNFα. Preferred methods for determining mAb specificity and affinity by competitive inhibition can be found in Muller, Meth. Enzymol. 92:589-601 (1983), which is entirely incorporated by reference.

As examples of antibodies according to the present invention, murine mAb A2 of the present invention is produced by a cell line designated c134A. Chimeric antibody cA2 is produced by a cell line designated c168A. Cell line c134A is deposited as a research cell bank in the Centocor Cell Biology Services Depository, and cell line c168A (RCB) is deposited as a research cell bank in the Centocor Corporate Cell Culture Research and Development Depository, both at Centocor, 200 Great Valley Parkway, Malvern, Pennsylvania, 19355. The c168A cell line is also deposited at Centocor BV, Leiden, The Netherlands.

Furthermore, c168A was deposited as of the filing date of the present application at the American Type Culture Collection, Rockville, Maryland, as a "Culture Safe Deposit."

The term "epitope" is meant to refer to that portion of any molecule capable of being recognized by and bound by an antibody. Epitopes usually consist of Chemically active surface groupings of molecules such as amino acids or sugar side chains and have specific three dimensional structural characteristics as well as specific charge characteristics. By "inhibiting and/or neutralizing epitope" is intended an epitope, which, when bound by an antibody, results in loss of biological activity of the molecule or organism containing the epitope, *in vivo, in vitro* and *in situ,* more preferably in *vivo,* including binding of TNF to a TNF receptor. Preferred antibodies, fragments and regions of anti-TNF antibodies of the present invention recognize epitopes including 5 amino acids comprising at least one amino acid from amino acids residues 87-108 or both residues 59-80 and 87-108 of hTNFα (of SEQ ID NO:1). Preferred antibodies, fragments and regions of anti-TNF antibodies of the present invention do not recognize epitopes from at least one of amino acids 11-13, 37-42, 49-57 or 155-157 of hTNFα (of SEQ ID NO:1). In a preferred embodiment, the epitope comprises at least 2 amino acids from residues 87-108 or both residues 59-80 and 87-108 of hTNFα (of SEQ ID NO:1). In another preferred embodiment, the epitope comprises at least 3 amino acids from residues 59-80 and 87-108 of hTNFα (of SEQ ID NO:1). In another preferred embodiment, the epitope comprises at least 4 amino acids from residues 87-108 or both residues 59-80 and 87-108 of hTNFα (of SEQ ID NO:1). In another preferred embodiment, the epitope comprises at least 5 amino acids from residues 67-108 or both residues 59-80 and 87-108 of bTNFα (of SEQ ID NO:1). In another preferred embodiment, the epitope comprises at least 6 amino acids from residues 87-108 or both residues 59-80 and 87-108 of hTNFα (of SEQ ID NO:1). In another preferred embodiment, the epitope comprises at least 7 amino acids from residues 87-108 or both residues 59-80 and 87-108 of hTNFα (of SEQ ID NO:1).

An "antigen" is a molecule or a portion of a molecule capable of being bound by an antibody which is additionally capable of inducing an animal to produce antibody capable of binding to an epitope of that antigen. An antigen may have one or more than one epitope. The specific reaction referred to above is meant to indicate that the antigen will react, in a highly selective manner, with its corresponding antibody and not with the multitude of other antibodies which may be evoked by other antigens. Preferred antigens that bind antibodies, fragments and regions of anti-TNF antibodies of the present invention include at least 5 amino acids comprising at least one of amino acids residues 87-108 or both residues 59-80 and 87-108 of hTNFα (of SEQ ID NO:1). Preferred antigens that bind antibodies, fragments and regions of anti-TNF antibodies of the present invention do not include amino acids of amino acids 11-13, 37-42, 49-57 or 155-157 of hTNFα (SEQ ID NO:1)

The term "antibody" is meant to include a polyclonal, or monoclonal antibody, and fragments and regions thereof, as well as derivatives thereof, which are capable of binding portions of TNF that inhibit binding to TNF receptors by TNF. Fragments include, for example, Fab, Fab', F(ab')₂ and Fv. These fragments lack the Fc fragment of intact antibody, clear more rapidly from the circulation, and may have less non-specific tissue binding than an intact antibody (Wahl et al, J. Nucl. Med. 24:316-325 (1983)). These fragments are produced from intact antibodies using methods well known in the art, for example by proteolytic cleavage with enzymes such as papain (to produce Fab fragments) or pepsin (to produce F(ab')₇ fragments). Regions of anti-TNF antibodies of the present invention include at least one of a heavy chain constant region (Hₒ), a heavy chain variable region (H,), a light chain variable region (Lᵥ) and a light chain constant regions (L_{c}), wherein a polyclonal, monoclonal antibody, fragments and regions thereof include at least one heavy chain variable region (Hᵥ) or light chain variable region (Lᵥ) which binds a portion of a TNF and inhibits TNF biological activity.

In a preferred embodiment, the antibody is a monoclonal antibody which binds amino acids of an epitope of TNF, which antibody is designated A2, rA2 or cA2, which is produced by a hybridoma or by a recombinant host. In another preferred embodiment, the antibody is a chimeric antibody which recognizes an epitope recognized by A2. In a more preferred embodiment, the antibody is a chimeric antibody designated as chimeric A2 (cA2). The murine and chimeric antibodies, fragments and regions of the present invention comprise individual heavy (H) and light (L) immunoglobulin chains. A chimeric H chain comprises an antigen binding region derived from the H chain of a non-human antibody specific for TNF, which is linked to at least a portion of a human H chain C region (C_{H}) .

A chimeric L chain according to the present invention, comprises an antigen binding region derived from the L chain of a non-human antibody specific for TNF, linked to at least a portion of a human L chain C region (C_{L}).

As used herein, the term "antigen binding region" refers to that portion of an antibody molecule which contains the amino acid residues that interact with an antigen and confer on the antibody its specificity and affinity for the antigen. The antibody region includes the "framework" amino acid residues necessary to maintain the proper conformation of the antigen-binding residues.

As used herein, the term "chimeric antibody" includes monovalent, divalent or polyvalent immunoglobulins. A monovalent chimeric antibody is a dimer (HL)) formed by a chimeric H chain associated through disulfide bridges with a chimeric L chain. A divalent chimeric antibody is tetramer (H₂L₂) formed by two HL dimers associated through at least one disulfide bridge. A polyvalent chimeric antibody can also be produced, for example, by employing a C_{H} region that aggregates (e.g., from an IgM H chain, or *µ* chain) .

The invention also provides for "derivatives" of the murine or chimeric antibodies, fragments, regions or derivatives thereof, which term includes those proteins encoded by truncated or modified genes to yield molecular species functionally resembling the immunoglobulin fragments. The modifications include, but are not limited to, addition of genetic sequences coding for cytotoxic proteins such as plant and bacterial toxins. The fragments and derivatives can be produced from any of the hosts of this invention. Alternatively, anti-TNF antibodies, fragments and regions can be bound to cytotoxic proteins or compounds in *vitro,* to provide cytotoxic anti-TNF antibodies which would selectively kill cells having TNF receptors.

Antibodies, fragments or derivatives having chimeric H chains and L chains of the same or different v region binding specificity, can be prepared by appropriate association of the individual polypeptide chains, as taught, for example by Sears et al., Proc. Natl. Acad. Sci. USA 72:353-357 (1975). With this approach, hosts expressing chimeric H chains (or their derivatives) are separately cultured from hosts expressing chimeric L chains (or their derivatives), and the immunoglobulin chains are separately recovered and then associated. Alternatively, the hosts can be co-cultured and the chains allowed to associate spontaneously in the culture medium, followed by recovery of the assembled immunoglobulin, fragment or derivative.

The antigen binding region of the chimeric antibody of the present invention is derived preferably from a non-human antibody specific for human TNF. Preferred sources for the DNA encoding such a non-human antibody include cell lines which produce antibody, preferably hybrid cell lines commonly known as hybridomas. A preferred hybridoma is the A2 hybridoma cell line. The hybrid cells are formed by the fusion of a non-human anti-hTNFα antibody-producing cell, typically a spleen cell of an animal immunized against either natural or recombinant human TNF, or a peptide fragment of the human TNFα protein sequence. Alternatively, the non-human anti-TNFα antibody-producing cell may be a B lymphocyte obtained from the blood, spleen, lymph nodes or other tissue of an animal immunized with TNF.

The antibody-producing cell contributing the nucleotide sequences encoding the antigen-binding region of the chimeric antibody of the present invention may also be produced by transformation of a non-human, such as a primate, or a human cell. For example, a B lymphocyte which produces anti-TNF antibody may be infected and transformed with a virus such as Epstein-Barr virus to yield an immortal anti-TNF producing cell (Kozbor et al. immunol. Today 4:72-79 (1983)). Alternatively, the B lymphocyte may be transformed by providing a transforming gene or transforming gene product, as is well-known in the art.

Preferably, the antigen binding region will be of murine origin. In other embodiments, the antigan binding region may be derived from other animal species, in particular rodents such as rabbit, rat or hamster.

The second fusion partner, which provides the immortalizing function, may be lymphoblastoid cell or a plasmacytoma or myeloma cell, which is not itself an antibody producing cell, but is malignant. Preferred fusion partner cells include the hybridoma SP2/0-Ag14, abbreviated as SP2/0 (ATCC CRL1581) and the myeloma P3X63Ag8 (ATCC TIB9), or its derivatives (see: Hartlow, E. et al., Antibodies: A Laboratory Manual, Cold Spring Harbor Laboratory Press, Cold Spring Harbor, NY, 1988)).

Marine hybridomas which produce mAb specific for human TNFα are formed by the fusion of a mouse fusion partner cell, such as SP2/0, and spleen cells from mice immunized against purified hTNFα, recombinant hTNFα, natural or synthetic TNF peptides, including peptides including 5 or more amino acids selected from residues 59-80, and 87-108 of TNF (of SBQ ID NO:1) or other biological preparations containing TNF. To immunize the mice, a variety of different conventional protocols may be followed. For example, mice may receive primary and boosting immunizations of TNF.

TNF residues 87-108 or both residues 59-80 and 87-108 of TNF (of SEQ ID NO:1), fragments or combinations of peptides containing therein are useful as immunogens to raise antibodies that will recognize peptide sequences presented in the context of the native TNF molecule.

Epitopes recognized by antibodies, and fragments and regions thereof, of the present invention include 5 or more amino acids comprising at least one amino acid of each or both of the following amino acid sequences of TNF, which provide a topographical epitope of TNF which is recognized by, and binds with anti-TNF activity, an antibody, and fragments, and variable regions thereof, of the present invention:

59-80:    Tyr-Ser-Gln-Val-Leu-Phe-Lys-Gly-Gln-Gly-Cys-
          Pro-Ser-Thr-His-Val-Leu-Leu-Thr-His-Thr-Ile
          (AA 59-80 of SEQ ID NO:1); and

87-108: Tyr-Gln-Thr-Lys-Val-Asn-Leu-Leu-Ser-Ala-Ile
-Lys-Ser-Pro-Cys-Gln-Arg-Glu-Thr-Pro-Glu-Gly
(AA 87-108 of SEQ ID NO:1).

particular peptides which can be used to generate antibodies of the present invention include combinations of amino acids selected from at least residues 87-108 or both residues 59-80 and 87-108, which are combined to provide an epitope of TNF that is bound by anti-TNF antibodies, fragments and regions thereof, and which binding provided anti-TNF biological activity. Such epitopes include at least 1-5 amino acids and less than 22 amino acids from residues 87-108 or each of residues 59-80 and 87-108, which in combination with other amino acids of TNF provide epitopes of at least 5 amino acids in length.

The techniques to raise antibodies of the present invention to small peptide sequences that recognize and bind to those sequences in the free or conjugated form or when presented as a native sequence in the context of a large protein are well known in the art. Such antibodies include murine, murine human and human-human antibodies produced by hybridoma or recombinant techniques known in the art.

The identification of these peptide sequences recognized by mAbs of the present invention provides the information necessary to generate additional monoclonal antibodies with binding characteristics and therapeutic utility that parallel the embodiments of this application.

In a preferred embodiment, the amino acids of the epitope are not of at least one of amino acids 11-13, 37-42, 49-57 and 155-157 of hTNFα (of SEQ ID NO:1).

The cell fusions are accomplished by standard procedures well known to those skilled in the field of immunology (Kohler and Milstein, Nature 256:495-497 (1975) and U.S. Patent No. 4,376,110; Hartlow, E. et al., supra; Campbell, A., "Monoclonal Antibody Technology," In: Laboratory Techniques in Biochemistry and Molecular Biology, Volume 13 (Burdon, R., et al., eds.), Elsevier, Amsterdam (1984); Kennett et al., Monoclonal Antibodies (Kennett et al., eds. pp. 365-367, Plenum Press, NY, 1980); de St. Groth, S.F., et al., J. Immunol. Meth. 35: 1-21 (1980); Galfre, G. et al., Methods Enzymol. 73:3-46 (1981); Goding, J.W. 1987. Monoclonal Antibodies: Principles and Practice. 2nd ed. Academic Press, London, 1987) ;

Fusion partner cell lines and methods for fusing and selecting hybridomas and screening for mAbs are well known in the art (Hartlow, E. et al., supra; Kawamoto, T.et al., Meth. Enzymol 121:266-277 (1986); Kearney, J.F. et al., J.Immunol. 123:1548-1550 (1979) : Kilmartin, J.V. et al., J. Cell Biol. 93:576-582 (1982); Kohler, G. et al., Eur. J. Immunol. 6:292-295 (1976); Lane, D.P. et al., J. Immunol. Meth. 47:303-307 (1981); Mueller, U.W. et al., J. Immunol. Meth. 87:193-196 (1986); Pontecorvo, G., Somatic Cell Genet. 1:397-400 (1975); Sharo, J., et al., Proc, Natl. Acad. Sci, USA 76:1420-1424 (1979); Shulman, M. et al., Nature 276:269-270 (1978); Springer, T.A. (ed), Hybridoma Technology in the Bioscienses and Medicine, plenum Press, New York, 1965; and Taggart, R.T. et al., Science 219:1228-1230 (1982)).

The hTNFα-specific marine or chimeric mAb of the present invention may be produced in large quantities by injecting hybridoma or transfectoma cells secreting the antibody into the peritoneal cavity of mice and, after appropriate time, harvesting the ascites fluid which contains a high titer of the mAb, and isolating the mAb therefrom. For such in vivo production of the mAb with a non-murine hybridoma (e.g., rat or human), hybridoma cells are preferably grown in irradiated or athymic nude mice.

Alternatively, the antibodies my be produced by culturing hybridoma or transfectoma cells in vitro and isolating secreted mAb from the cell culture medium.

Monoclonal antibodies of the present invention recognize epitopes including non-contiguous residues located within the non-contiguous sequences residues 87-108 or both residues 59-80 and 87-108 of TNF (of SEQ ID NO:1). Preferred anti-TNF mAbs are those that inhibit this binding of human TNF-α to its receptors by virtue of their ability to bind to one or more of these peptide sequences. These antibodies would block the activity of TNF by virtue of binding to the epitope of sequences including 87-108 and/or 110-128 of TNF (of SBQ ID NO:1) . Such binding is demonstrated to inhibit TNF activity, as described herein.

Particular peptides which can be used to screen antibodies of the present invention include combinations of amino acids selected from at least residues 87-108 or both residues 59-80 and 87-108, which are combined to provide an epitope of TNF that is bound by anti-TNF antibodies, fragments and regions thereof, of the present invention, and which binding provided anti-TNF biological activity. Such epitopes include at least 1-5 amino acids and less than 22 amino acids from residues 87-108 or each of residues 59-80 and 87-108, which in combination with other amino acids of TNF provide epitopes of at least 5 amino acids in length.

### Recombinant Expression of anti-TNF Activity Antibodies

Recombinant murine or chimeric murine-human or human-human antibodies that inhibit TNF and bind an epitope included in the amino acid sequences residues 87-108 or both residues 59-80 and 87-108 of hTNFα (of SEQ ID NO:1), can be provided according to the present invention using known techniques based on the teaching provided herein.

The DNA encoding an anti-TNF antibody of the present invention may be genomic DNA or cDNA which encodes at least one of the heavy chain constant region (Hₒ), the heavy chain variable region (Hy), the light chain variable region (Lᵥ) and the light chain constant regions (Lₒ). A convenient alternative to the use of chromosomal gene fragments as the source of DNA encoding the murine V region antigen-binding segment is the use of cDNA for the construction of chimeric immunoglobulin genes, as reported by Liu et al. (Proc. Natl. Acad. Sci., USA .84:3439 (1987) and J. Immunology 139:3521 (1987), which references are hereby entirely incorporated by reference. The use of cDNA requires that gene expression elements appropriate for the host cell be combined with the gene in order to achieve synthesis of the desired protein. The use of cDNA sequences is advantageous over genomic sequences (which contain introns), in that cDNA sequences can be expressed in bacteria or other hosts which lack appropriate RNA splicing systems.

Human genes which encode the constant (C) regions of the marine and chimeric antibodies, fragments and regions of the present invention can be derived from a human fetal liver library, by known methods. Human C regions genes may be derived from any human cell including those which express and produce human immunoglobulins. The human C_{H} region can be derived from any of the known classes or isotypes of human H chains, including gamma, *µ*, α, **δ** or ε, and subtypes thereof, such as G1, G2, G3 and G4. Since the H chain isotype is responsible for the various effector functions of an antibody, the choice of C_{H} region will be guided by the desired effector functions, such as complement fixation, or activity in antibody-dependent cellular cytotoxicity (ADCC) - Preferably, the C_{H} region is derived from gamma 1 (IgG1), gamma 3 (IgG3), gamma 4 (IgG4), or µ (IgM).

The human C_{L} region can be derived from either human L chain isotype, kappa or lambda.

Genes encoding human immunoglobulin C regions are obtained from human cells by standard cloning techniques (Sambrook, J. et al. (Molecular ,Cloning : A Laboratory Manual, 2nd Edition, Cold Spring Harbor Press, Cold Spring Harbor, NY (1989) and Ausubel et al, eds. Current Protocols in Molecular Biology (1987-1991)). Human C region genes are readily available from known clones containing genes representing the two classes of L chains, the five classes of H chains and subclasses thereof. Chimeric antibody fragments, such as F(ab')₂ and Fab, can be prepared by designing a chimeric H chain gene which is appropriately truncated. For example, a chimeric gene encoding an H chain portion of an F(ab')₂ fragment would include DNA sequences encoding the CH₁ domain and hinge region of the H Chain, followed by a translational stop codon to yield the truncated molecule.

Generally, the murine, human or murine and chimeric antibodies, fragments and regions of the present invention are produced by cloning DNA segments encoding the H and L chain antigen-binding regions of a TNF-specific antibody, and joining these DNA segments to DNA segments encoding C_{H} and C_{L} regions, respectively, to produce murine, human or chimeric immunoglobulin-encoding genes.

Thus, in a preferred embodiment, a fused chimeric gene is created which comprises a first DNA segment that encodes at least the antigen-binding region of non-human origin, such as a functionally rearranged V region with joining (J) segment, linked to a second DNA segment encoding at least a part of a human C region.

Therefore, cDNA encoding the antibody V and C regions, the method of producing the chimeric antibody according to the present invention involves several steps, outlined below:
1. isolation of messenger RNA (mRNA) from the cell line producing an anti-TNF antibody and from optional additional antibodies supplying heavy and light constant regions; cloning and cDNA production therefrom;
2. preparation of a full length cDNA library from purified mRNA from which the appropriate V and/or C region gene segments of the L and H chain genes can be: (i) identified with appropriate probes, (ii) sequenced, and (iii) made compatible with a C or V gene segment from another antibody for a chimeric antibody;
3. Construction of complete H or L chain coding sequences by linkage of the cloned specific V region gene segments to cloned C region gene, as described above;
4. Expression and production of L and H chains in selected hosts, including prokaryotic and eukaryotic cells to provide murine-murine, human-murine, human-human or human murine antibodies.

One common feature of all immunoglobulin H and L chain genes and their encoded mRNAs is the J region. H and L chain J regions have different sequences, but a high degree of sequence homology exists (greater than 80%) among each group, especially near the C region. This homology is exploited in this method and consensus sequences of H and L chain J regions may be used to design oligonucleotides for use as primers for introducing useful restriction sites into the J region for subsequent linkage of V region segments to human C region segments.

C region cDNA vectors prepared from human cells can be modified by site-directed mutagenesis to place a restriction site at the analogous position in the human sequence. For example, one can clone the complete human kappa chain C (Cₜ) region and the complete human gamma-1 C region (C_{gamma-1}). In this case, the alternative method based upon genomic C region clones as the source for C region vectors would not allow these genes to be expressed in bacterial systems where enzymes needed to remove intervening sequences are absent. Cloned v region segments are excised and ligated to L or B chain C region vectors. Alternatively, the human C_{gamma-1} region can be modified by introducing a termination codon thereby generating a gene sequence which encodes the H chain portion of an Fab molecule. The coding sequences with linked V and C regions are then transferred into appropriate expression vehicles for expression in appropriate hosts, prokaryotic or eukaryotic.

Two coding DNA sequences are said to be "operably linked" if the linkage results in a continuously translatable sequence without alteration or interruption of the triplet reading frame. A DNA coding sequence is operably linked to a gene expression element if the linkage results in the proper function of that gene expression element to result in expression of the coding sequence.

Expression vehicles include plasmids or other vectors. Preferred among these are vehicles carrying a functionally complete human C_{H} or C_{L} chain sequence having appropriate restriction sites engineered so that any V_{H} or V_{L} chain sequence with appropriate cohesive ends can be easily inserted therein. Human C_{H} or C_{L} chain sequence-containing vehicles thus serve as intermediates for the expression of any desired complete H or L chain in any appropriate host.

A chimeric antibody, such as a mouse-human or human-human, will typically be synthesized from genes driven by the chromosomal gene promoters native to the mouse H and L chain V regions used in the constructs; splicing usually occurs between the splice donor site in the mouse J region and the splice acceptor site preceding the human C region and also at the splice regions that occur within the human C_{H} region; polyadenylation and transcription termination occur at native chromosomal sites downstream of the human coding regions.

Gene expression elements useful for the expression of cDNA genes include: (a) viral transcription promoters and their enhancer elements, such as the SV40 early promoter (Okayama, H. et al, Mol, Cell, Biol. 3:280 (1963)), Rous sarcoma virus LTR (Gorman, C. et al., Proc. Natl. Acad, Sci.,USA 79:6777(1982)), and Moloney murine leukemia virus LTR (Grosseched1, R.et al., Cell 41:885 (1985)); (b) splice regions and polyadenylation sites such as those derived from the SV40 late region (Okayama et al., supra); and (c) polyadenylation sites such as in SV40 (Okayama et al., supra).

Immunoglobulin cDNA genes may be expressed as described by Liu et al., supra, and Weidle et al., Gene 51:21 (1987), using as expression elements the SV40 early promoter and its enhancer, the mouse immunoglobulin H chain promoter enhancers, SV40 late region mRNA splicing, rabbit β-globin intervening sequence, immunoglobulin and rabbit β-globin polyadenylation sites, and SV40 polyadenylation elements. For immunoglobulin genes comprised of part cDNA, part genomic DNA (Whittle et al., Protein Engineering 1:499 (1987)), the transcriptional promoter is human cytomegalovirus, the promoter enhancers are cytomegalovirus and mouse/human immunoglobulin, and mRNA splicing and polyadenylation regions are from the native chromosomal immunoglobulin sequences.

In one embodiment, for expression of cDNA genes in rodent cells, the transcriptional promoter is a viral LTR sequence, the transcriptional promoter enhancers are either or both the mouse immunoglobulin heavy chain enhancer and the viral LTR enhancer, the splice region contains an intron of greater than 31 bp, and the polyadenylation and transcription termination regions are derived from the native chromosomal sequence corresponding to the immunoglobulin chain being synthesized. In other embodiments, cDNA sequences encoding other proteins are combined with the above-recited expression elements to achieve expression of the proteins in mammalian cells.

Each fused gene is assembled in, or inserted into, an expression vector. Recipient cells capable of expressing the chimeric immunoglobulin chain gene product are then transfected singly with a chimeric H or chimeric L chain-encoding gene, or are co-transfected with a chimeric H and a chimeric L chain gene. The transfected recipient cells are cultured under conditions that permit expression of the incorporated genes and the expressed immunoglobulin chains or intact antibodies or fragments are recovered from the culture.

In one embodiment, the fused genes encoding the chimeric E and L chains, or portions thereof, are assembled in separate expression vectors that are then used to co-transfect a recipient cell.

Each vector may contain two selectable genes, a first selectable gene designed for selection in a bacterial system and a second selectable gene designed for selection in a eukaryotic system, wherein each vector has a different pair of genes. This strategy results in vectors which first direct the production, and permit amplification, of the fused genes in a bacterial system. The genes so produced and amplified in a bacterial host are subsequently used to **co-transfect** a eukaryotic cell, and allow selection of a co-transfected cell carrying the desired transfected genes.

Examples of selectable genes for use in a bacterial system are the gene that confers resistance to ampicillin and the gene that confers resistance to chloramphenicol. Preferred selectable genes for use in eukaryotic transfectants include the xanthine guanine phosphoribosyl transferase gene (designated gpt.) and the phosphotransferase gene from Tn5 (designated neo). Selection of cells expressing gpt is based on the fact that the enzyme encoded by this gene utilizes xanthine as a substrate for purine nucleotide synthesis, whereas the analogous endogenous enzyme cannot. In a medium containing (1) mycophenolic acid, which blocks the conversion of inosine monophosphate to xanthine monophosphate, and (2) xanthine, only cells expressing the gpt gene can survive. The product of the neo blocks the inhibition of protein synthesis by the antibiotic G418 and other antibiotics of the neomycin class.

The two selection procedures can be used simultaneously or sequentially to select for the expression of immunoglobulin chain genes introduced on two different DNA. vectors into a eukaryotic cell. It is not necessary to include different selectable markers for eukaryotic cells; an H and an L chain vector, each containing the same selectable marker can be co-transfected. After selection of the appropriately resistant cells, the majority of the clones will contain integrated copies of both H and L chain vectors.

Alternatively, the fused genes encoding the chimeric H and L chains can be assembled on the same expression vector.

For transfection of the expression vectors and production of the chimeric antibody, the preferred recipient cell line is a myeloma cell. Myeloma cells can synthesize, assemble and secrete immumoglobulins encoded by transfected immunoglobulin genes and possess the mechanism for glycosylation of the immunoglobulin. A particularly preferred recipient cell is the Ig-non-producing myeloma cell SP2/0 (ATCC #CRL 8287). SP2/0 cells produce only immunoglobulin encoded by the transfected genes. Myeloma cells can be grown in culture or in the peritoneal cavity of a mouse, where secreted immunoglobulin can be obtained from ascites fluid. Other suitable recipient cells include lymphoid cells such as B lymphocytes of human or non-human origin, hybridoma cells of human or non-human origin, or interspecies heterohybridoma cells.

The expression vector carrying a chimeric antibody construct of the present invention may be introduced into an appropriate host cell by any of a variety of suitable means, including such biochemical means as transformation, transfection, conjugation, protoplast fusion, calcium phosphate-precipitation, and application with polycations such as diethylaminoethyl (DEAE) dextran, and such mechanical means as electroporation, direct microinjection, and microprojectile bombardment (Johnston et al., Science 240;1538 (1988)). A preferred way of introducing DNA into lymphoid cells is by electroporation (Potter et al., Proc. Natl. Acad. Sci. USA 81:7161 (1984); Yoshikawa. K. et al., Jpn. J. Cancer Res. 77: 1122-1133). In this procedure, recipient cells are subjected to an electric pulse in the presence of the DNA to be incorporated. Typically, after transfection, cells are allowed to recover in complete medium for about 24 hours, and are then seeded in 96-well culture plates in the presence of the selective medium. G418 selection is performed using about 0.4 to 0.8 mg/ml 0418. Mycophenolic acid selection utilizes about 6*µ*g/ml plus about 0.25 mg/ml xanthine. The electroporation technique is expected to yield transfection frequencies of about 10⁻³ to about 10⁻⁴ for Sp2/0 cells. In the protoplast fusion method, lysozyme is used to strip cell walls from catarrhal harboring the recombinant plasmid containing the chimeric antibody gene. The resulting spheroplasts are fused with myeloma cells with polyethylene glycol.

The immunoglobulin genes of the present invention can also be expressed in nonlymphoid mammalian cells or in other eukaryotic cells, such as yeast, or in prokaryotic cells, in particular bacteria.

Yeast provides substantial advantages over bacteria for the production of immunoglobulin H and L chains. Yeasts carry out post-translational peptide modifications including glycosylation. A number of recombinant DNA strategies now exist which utilize strong promoter sequences and high copy number plasmids which can be used for production of the desired proteins in yeast. Yeast recognizes leader sequences of cloned mammalian gene products and secretes peptides bearing leader sequences (i.e., pre-peptides) (Hitzman, et al., 11th International Conference on Yeast. Genetics and Molecular Biology, Montpellier, FranCe, September 13-17, 1982).

Yeast gene expression systems can be routinely evaluated for the levels of production, secretion and the stability of chimeric H and L chain proteins and assembled murine and chimeric antibodies, fragments and regions . Any of a series of yeast gene expression **systems** incorporating promoter and termination elements from the actively expressed genes coding for glycolytic enzymes produced in large quantities when yeasts are grown in media rich in glucose can be utilized. Known glycolytic genes can also provide very efficient transcription control signals. For example, the promoter and terminator signals of the phosphoglycerate kinase (PGK) gene can be utilized. A number of approaches may be taken for evaluating optimal expression plasmids for the expression of cloned immunoglobulin cDNAs in yeast (see Glover, D.M., ed., DNA Cloning, Vol. II, pp45-66, IRL Press, 1985).

Bacterial strains may also be utilized as hosts for the production of antibody molecules or antibody fragments described by this invention, E. coli K12 strains such as E. coli W3110 (ATCC 27325), and other enterobacteria such as Salmonella typhimurium or serratia marcescens, and various Pseudomonas species may be used.

Plasmid vectors containing replicon and control sequences which are derived from species compatible with a host cell are used in connection with these bacterial hosts. The vector carries a replication site, as well as specific genes which are capable of providing phenotypic selection in transformed cells. A number of approaches may be taken for evaluating the expression plasmids for the production of murine and chimeric antibodies, fragments and regions or antibody chains encoded by the cloned immunoglobulin cDNAs in bacteria (see Glover, D.M., ed., DNA Cloning. Vol. I, IRI. Press, 1985).

Preferred hosts are mammalian cells, grown in vitro or in vivo. Mammalian cells provide post- translational modifications to immunoglobulin protein molecules including leader peptide removal, folding and assembly of H and L chains, glycosylation of the antibody molecules, and secretion of functional antibody protein.

Mammalian cells which may be useful as hosts for the production of antibody proteins, in addition to the cells of lymphoid origin described above, include cells of fibroblast origin, such as Vero (ATCC CRL 81) or CHO-K1 (ATCC CRL 61).

Many vector systems are available for the expression of cloned H and L chain genes in mammalian cells (see Glover, D.M., ed., DNA Cloning, Vol. II, pp143-238, IRL Press, 1985). Different approaches can be followed to obtain complete H₂L₂ antibodies. As discussed above, it is possible to co-express H and L chains in the same cells to achieve intracellular association and linkage of B and L chains into complete tetrameric H₂L₂ antibodies. The co-expression can occur by using either the same or different plasmids in the same host. Genes for both H and L chains can be placed into the same plasmid, which is then transfected into cells, thereby selecting directly for cells that express both chains. Alternatively, cells may be transfected first with a plasmid encoding one chain, for example the L chain, followed by transfection of the resulting cell line with an H chain plasmid containing a second selectable marker. Cell lines producing H₂L₂ molecules via either route could be transfected with plasmids encoding additional copies of H, L, or H plus L chains in conjunction with additional selectable markers to generate cell lines with enhanced properties, such as higher production of assembled H₂L₂ antibody molecules or enhanced stability of the transfected cell lines.

In addition to monoclonal or chimeric anti-TNF antibodies, the present invention is also directed to an anti-idiotypic (anti-Id) antibody specific for the anti-TNF antibody of the invention. An anti-Id antibody is an antibody which recognizes unique determinants generally associated with the antigen-binding region of another antibody. The antibody specific for TNF is termed the idiotypic or Id antibody. The anti-Id can be prepared by immunizing an animal of the same species and genetic type (e.g. mouse strain) as the source of the Id antibody with the Id antibody or the antigen-binding region thereof. The immunized animal will recognize and respond to the idiotypic determinants of the immunizing antibody and produce an anti-Id antibody. The anti-Id antibody may also be used as an "immunogen" to induce an immune response in yet another animal, producing a so-called anti-anti-Id antibody. The anti-anti-Id may be epitopically identical to the original antibody which induced the anti-Id. Thus, by using antibodies to the idiotypic determinants of a mAb, it is possible to identify other clones expressing antibodies of identical specificity.

Accordingly, mAbs generated against TNF according to the present invention may be used to induce anti-Id antibodies in suitable animals, such as BALB/c mice. Spleen cells from such immunized mice can be used to produce anti-Id hybridomas secreting anti-Id mAbs. Further, the anti-Id mAbs can be coupled to a carrier such as keyhole limpet hemocyanin (KLH) and used to immunize additional BALB/c mice. Sera from these mice will contain anti-anti-Id antibodies that have the binding properties of the original mAb specific for a TNF epitope.

The antibodies, fragments and derivatives of the present invention are useful for treating a subject having a pathology or condition associated with levels of a substance reactive with an anti-TNF antibody, in particular TNF, in excess of the levels present in a normal healthy subject. Such pathologies include, but are not limited to, sepsis syndrome, including cachexia, circulatory collapse and shock resulting from acute or chronic bacterial infection, acute and chronic parasitic or infectious processes, including bacterial, viral and fungal infections, acute and chronic immune and autoimmune pathologies, such as systemic lupus erythematosus and rheumatoid arthritis, alcohol-induced hepatitis, chronic inflammatory, pathologies such as sarcoidosis and Crohn's pathology, vascular inflammatory pathologies such as disseminated intravascular coagulation, graft-versus-host pathology, Kawasaki's pathology and malignant pathologies involving TNF-secreting tumors.

Such treatment comprises parenterally administering a single or multiple doses of the antibody, fragment or derivative. Preferred for human pharmaceutical use are high affinity potent hTNFα:-inhibiting and/or neutralizing murine and chimeric antibodies, fragments and regions of this invention.

Monoclonal antibodies may be administered by any means that enables the active agent to reach the agent's site of action in the body of a mammal. In the case of the antibodies of this invention, the primary focus is the ability to reach and bind with TNF released by monocytes and macrophages. Because proteins are subject to being digested when administered orally, parenteral administration, i.e., intravenous, subcutaneous, intramuscular, would ordinarily be used to optimize absorption.

Monoclonal antibodies may be administered either as individual therapeutic agents or in combination with other therapeutic agents. They can be administered alone, but are generally administered with a pharmaceutical carrier selected on the basis of the chosen route of administration and standard pharmaceutical practice.

The dosage administered will, of course, vary depending upon known factors such as the pharmacodynamic characteristics of the particular agent, and its mode and route of administration; age, health, and weight of the recipient; nature and extent of symptoms, kind of concurrent treatment, frequency of treatment, and the effect desired. Usually a daily dosage of active ingredient can be about 0.1 to 100 milligrams per kilogram of body weight. Ordinarily 0.5 to 50, and preferably 1 to 10 milligrams per kilogram per day given in divided doses 1 to 6 times a day or in sustained release form is effective to obtain desired results.

Dosage forms (composition) suitable for internal administration generally contain from about 1 milligram to about 500 milligrams of active ingredient per unit. In these pharmaceutical compositions the active ingredient will ordinarily be present in an amount of about 0.5-95% by weight based on the total weight of the composition.

For parenteral administration, the antibody can be formulated as a solution, suspension, emulsion or lyophilized powder in association with a pharmaceutically acceptable parenteral vehicle. Examples of such vehicles are water, saline, Ringer's solution, dextrose solution, and 5% human serum albumin. Liposomes and nonaqueous vehicles such as fixed oils may also be used. The vehicle or lyophilized powder may contain additives that maintain isotonicity (e.g., sodium chloride, mannitol) and chemical stability (e.g., buffers and preservatives). The formulation is sterilized by commonly used techniques.

Suitable pharmaceutical carriers are described in the most recent edition of Remington's Pharmaceutical Sciences, A. Osol, a standard reference text in this field.

For example, a parenteral composition suitable for administration by injection is prepared by dissolving 1.5% by weight of active ingredient in 0.9% sodium chloride solution.

the antibodies of this invention can be adapted for therapeutic efficacy by virtue of their ability to mediate antibody-dependent cellular cytotoxicity (ADCC) and/or complement-dependent cytotoxicity (CDC) against cells having TNF associated with their surface. For these activities, either an endogenous source or an exogenous source of effector cells (for ADCC) or complement components (for CDC) can be utilized. The murine and chimeric antibodies, fragments and regions of this invention, their fragments, and derivatives can be used therapeutically as immunoconjugates (see for review: Dillman, R.O., Ann. Int. Med, 111:592-603 (1989)). They can be coupled to cytotoxic proteins, including, but not limited to Ricin-A, Pseudomonas toxin, Diphtheria toxin, and TNF. Toxins conjugated to antibodies or other ligands, are known in the art (see, for example, Olsnes, S. et al., Immunol. Today 10:291-295 (1989)). Plant and bacterial toxins typically kill cells by disrupting the protein synthetic machinery.

The antibodies of this invention can be conjugated to additional types of therapeutic moieties including, but not limited to, radionuclides, cytotoxic agents and drugs. Examples of radionuclides which can be coupled to antibodies and delivered in vitro to sites of antigen include ²¹²Bi, ¹³¹I, ¹⁸⁶Re, and ⁹⁰Y, which list is not intended to be exhaustive. The radionuclides exert their cytotoxic effect by locally irradiating the cells, leading to various intracellular lesions, as is known in the art of radiotherapy.

Cytotoxic drugs which can be conjugated to antibodies and subsequently used for in vivo therapy include, but are not limited to, daunorubicin, doxorubicin, methotrexate, and Mitomycin C. Cytotoxic drugs interfere with critical cellular processes including DNA, RNA, and protein synthesis. For a fuller exposition of these classes of drugs which are known in the art, and their mechanisms of action, see Goodman, A.G., et al., Goodman and Gilman's THE PHARMACOLOGICAL BASIS OF THERAPEUTICS, 7th Ed., Macmillan Publishing Co., 1985.

The antibodies of this invention may be advantageously utilized in combination with other monoclonal or murine and chimeric antibodies, fragments and regions, or with lymphokines or hemopoietic growth factors, etc., which serve to increase the number or activity of effector cells which interact with the antibodies.

The antibodies, fragments or derivatives of this invention may also be used in combination with TNF therapy to block undesired side effects of 7NF. Recent approaches to cancer therapy have included direct administration of TNF to cancer patients or immunotherapy of cancer patients with lymphokine activated killer (LAK) cells (Rosenberg et al., New Eng. J. Med. 313:1485-1492 (1985)) or tumor infiltrating lymphocytes (TIL) (Kurnick et al. (Clin. Immunol. Immunopath. 38:367-380 (1986); Kradin et al., Cancer Immunol. Immunother. 24:76-85 (1987) ; Kradin et al., Transplant. Proc. 20:336-338 (1988)). Trials are currently underway using modified LAK cells or TIL which have been transfected with the TNF gene to produce large amounts of TNF. Such therapeutic approaches are likely to be associated with a number of undesired side effects caused by the pleiotropic actions of TNF which were described above. According to the present invention, these side effects can be reduced by concurrent treatment of a subject receiving TNF or cells producing large amounts of TIL with the antibodies, fragments or derivatives of the present invention. Effective doses are as described above. The dose level will require adjustment according to the dose of TNF or TNF-producing cells administered, in order to block side effects without blocking the main anti-tumor effect of TNF. One of ordinary skill in the art will know how to determine such doses without undue experimentation.

The murine and chimeric antibodies, fragments and regions, fragments, or derivatives of this invention, attached to a solid support, can be used to remove TNF from fluids or tissue or cell extracts. In a preferred embodiment, they are used to remove TNF from blood or blood plasma products. In another preferred embodiment, the murine and chimeric antibodies, fragments and regions are advantageously used in extracorporeal immunoadsorbent devices, which are known in the art (see, for example, Seminars in Hematology, Vol. 26 (2 Suppl. 1) (1989)). Patient blood or other body fluid is exposed to the attached antibody, resulting in partial or complete removal of circulating TNF (free or in immune complexes), following which the fluid is returned to the body. This immunoadsorption can be implemented in a continuous flow arrangement, with or without interposing a cell centrifugation step. See, for example, Terman, D.S. et. al., J. Immunol. 117:1971-1975 (1976).

The present invention also provides the above antibodies, fragments and derivatives, detectably labeled, as described below, for use in diagnostic methods for detecting TNFα in patients known to be or suspected of having a TNFα-mediated condition.

The antibodies of the present invention are useful for immunoassays which detect or quantitate TNF, or anti-TNF antibodies, in a sample. An immmoassay for TNF typically comprises incubating a biological sample in the presence of a detectably labeled high affinity antibody of the present invention capable of selectively binding to TNF, and detecting the labeled antibody which is bound in a sample. Various clinical immunoassay procedures are described in Immunoassays for the 80' s, A. Voller et al., eds., University Park, 1981.

Thus, in this aspect of the invention, the antibody or a biological sample may be added to nitrocellulose, or other solid support which is capable of immobilizing cells, cell particles or soluble proteins. The support may then be washed with suitable buffers followed by treatment with the detectably labeled TNF-specific antibody. The solid phase support may then be washed with the buffer a second time to remove unbound antibody. The amount of bound label on said solid support may then be detected by conventional means.

By "solid phase support" or "carrier" is intended any support capable of binding antigen or antibodies. Well-known supports or carriers, include glass, polystyrene, polypropylene, polyethylene, dextran, nylon, amylases, natural and modified celluloses, polyacrylamides, agaroses, and magnetite. The nature of the carrier can be either soluble to some extent or insoluble for the purposes of the present invention. The support material may have virtually any possible structural configuration so long an the coupled molecule is capable of binding to TNF or an anti-TNP antibody. Thus, the support configuration may be spherical, as in a bead, or cylindrical, as in the inside surface of a test tube, or the external surface of a rod. Alternatively, the surface may be flat such as a sheet, test strip, etc. Preferred supports include polystyrene beads. Those skilled in the art will know many other suitable carriers for binding antibody or antigen, or will be able to ascertain the same by use of routine experimentation.

The binding activity of a given lot of anti-TNF antibody may be determined according to well known methods. Those skilled in the art will be able to determine operative and optimal assay conditions for each determination by employing routine experimentation.

one of the ways in which the THF-specific antibody can be detectably labeled is by linking the same to an enzyme and use in an enzyme immunoassay (EIA), or enzyme-linked inmunosorbent assay (ELISA). This enzyme, when subsequently exposed to its
substrate, will react with the substrate generating a chemical moiety which can be detected, for example, by spectrophotometric, fluorometric or by visual means. Enzymes which can be used to detectably label the TNF-specific antibodies of the present invention include, but are not limited to, malate dehydrogenase, staphylococcal nuclease, delta-5-steroid isomerase, yeast alcohol dehydrogenase, alpha-glycerophosphate dehydrogenase, triose phosphate isomerase, horseradish peroxidase, alkaline phosphatase, asparaginase, glucose oxidase, beta-galactosidase, ribonuclease, urease, catalase, glucose-6-phosphate dehydrogenase, glucoamylase and acetylcholinesterase.

By radioactively labeling the TNF-specific antibodies, it is possible to detect TNF through the use of a radioimmunoassay (RIA) (see, for example, Work, T.S., et al., Laboratory Techniques and Biochemistry in Molecular Biology, North Holland Publishing Company, N.V. (1978). The radioactive isotope can be detected by such means as the use of a gamma counter or a scintillation counter or by autoradiography. Isotopes which are particularly useful for the purpose of the present invention are: ⁹H, ¹²⁹I, ¹³¹I, ³⁵S, ¹⁴C, and, preferably, ¹²¹I.

It is also possible to label the TNF-specific antibodies with a fluorescent compound. When the fluorescent labeled antibody is exposed to light of the proper wave length, its presence can then be detected due to fluorescence. Among the most commonly used fluorescent labelling compounds are fluorescein isothiocyanate, rhodamine, phycoerythrin, phycocyanin, allophycocyanin, Q-phthaldayde and fluorescamine.

The TNF-specific antibodies can also be detectably labeled using fluorescence-emitting metals such as ¹⁵²Eu, or others of the lanthanide series. These metals can be attached to the TNF-specific antibody using such metal chelating groups as diethylenetriaminepentaacetic acid (DTPA) or ethylenediamine-tetraacetic acid (EDTA).

The TNF-specific antibodies also can be detectably labeled by coupling to a chemiluminescent compound. The presence of the chemiluminescently labeled antibody is then determined by detecting the presence of luminescence that arises during the course of a chemical reaction. Examples of particularly useful chemiluminescent labeling compounds are luminol, isoluminol, theromatic acridinium ester, imidazole, acridinium salt and oxalate ester.

Likewise, a bioluminescent compound may be used to label the TNF-specific antibody, fragment or derivative of the present invention. Bioluminescence is a type of chemiluminescence found in biological systems in which a catalytic protein increases the efficiency of the chemiluminescent reaction. The presence of a bioluminescent protein is determined by detecting the presence of luminescence. Important bioluminescent compounds for purposes of labeling are luciferin, luciferase and aequorin.

Detection of the TNF-specific antibody, fragment or derivative may be accomplished by a scintillation counter, for example, if the detectable label is a radioactive gamma emitter, or by a fluorometer, for example, if the label is a fluorescent material. In the case of an enzyme label, the detection can be accomplished by colorometric methods which employ a substrate for the enzyme. Detection may also be accomplished by visual comparison of the extent of enzymatic reaction of a substrate in comparison with similarly prepared standards.

For the purposes of the present invention, the TNF which is detected by the above assays may be present in a biological sample. Any sample containing TNF can be used. Preferably, the sample is a biological fluid such as, for example, blood, serum, lymph, urine, inflammatory exudate, cerebrospinal fluid, amniotic fluid, a tissue extract or homogenate, and the like. However, the invention is not limited to assays using only these samples, it being possible for one of ordinary skill in the art to determine suitable conditions which allow the use of other samples.

In situ detection may be accomplished by removing a histological specimen from a patient, and providing the combination of labeled antibodies of the present invention to such a specimen. The antibody (or fragment) is preferably provided by applying or by overlaying the labeled antibody (or fragment) to a biological sample. Through the use of such a procedure, it is possible to determine not only the presence of TNF but also the distribution of TNF in the examined tissue. Using the present invention, those of ordinary skill will readily perceive that any of a wide variety of histological methods (such as staining procedures) can be modified in order to achieve such in situ detection.

The antibody, fragment or derivative of the present invention may be adapted for utilization in an immmometric assay, also known as a "two-site" or "sandwich" assay. In a typical immunometric assay, a quantity of unlabeled antibody (or fragment of antibody) is bound to a solid support that is insoluble in the fluid being tested and a quantity of detectably labeled soluble antibody is added to permit detection and/or quantitation of the ternary complex formed between solid-phase antibody, antigen, and labeled antibody.

Typical, and preferred, immunometric assays include "forward" assays in which the antibody bound to the solid phase is first contacted with the sample being tested to extract the TNF from the sample by formation of a binary solid phase antibody-TNF complex. After a suitable incubation period, the solid support is washed to remove the residue of the fluid sample, including unreacted TNF, if any, and then contacted with the solution containing a known quantity of labeled antibody (which functions as a "reporter molecule"). After a second incubation period to permit the labeled antibody to complex with the TNF bound to the solid support through the unlabeled antibody, the solid support is washed a second time to remove the unreacted labeled antibody. This type of forward sandwich assay may be a simple "yes/no" assay to determine whether TNF is present or may be made quantitative by comparing the measure of labeled antibody with that obtained for a standard sample containing known quantities of TNF. Such "two-site" or "sandwich" assays are described by Wide (Radioimmune Assay Method, Kirkham, ed., E. & S. Livingstone, Edinburgh, 1970, pp. 199-206).

Other type of "sandwich" assays, which may also be useful with TNF, are the so-called "simultaneous" and "reverse" assays. A simultaneous assay involves a single incubation step wherein the antibody bound to the solid support and labeled antibody are both added to the sample being tested at the same time. After the incubation is completed, the solid support is washed to remove the residue of fluid sample and uncomplexed labeled antibody. The presence of labeled antibody associated with the solid support is then determined as it would be in a conventional "forward" sandwich assay.

In the "reverse" assay, stepwise addition first of a solution of labeled antibody to the fluid sample followed by the addition of unlabeled antibody bound to a solid support after a suitable incubation period, is utilized. After a second incubation, the solid phase is washed in conventional fashion to free it of the residue of the sample being tested and the solution of unreacted labeled antibody. The determination of labeled antibody associated with a solid support is then determined as in the "simultaneous" and "forward" assays. In one embodiment, a combination of antibodies of the present invention specific for separate epitopes may be used to construct a sensitive three-site immunoradiometric assay.

Having now generally described the invention, the same will be further understood by reference to certain specific examples which are included herein for purposes of illustration only and are not intended to be limiting unless otherwise specified.

### EXAMPLE I

### Production a Mouse Anti-Human THF mAb

To facilitate clinical study of TNF mAb a high-affinity potent inhibiting and/or neutralizing mouse anti-human TNF IgG1 mAb designated A2 was produced. Female BALB/c mice, 10 weeks old, were obtained from the Jackson Laboratory (Bar Harbor, ME). Forty µg of purified E. coli-derived recombinant human TNF (rhTNF) emulsified with an equal volume of complete Freund's adjuvant (obtained from Difco Laboratories) in 0.4 ml was injected subcutaneously and intraperitoneally (i.p.) into a mouse. One week later, an injection of 5 *µ*g of rhTNF in incomplete Freund's adjuvant was given i.p. followed by four consecutive i.p. injections of 10 *µ*g of TNF without adjuvant. Eight weeks after the last injection, the mouse was boosted i.p. with 10 *µ*g of TNF.

Four days later, the mouse was sacrificed, the spleen was obtained and a spleen cell suspension was prepared. Spleen cells were fused with cells of the nonsecreting hybridoma, Sp2/0 (ATCC CRL1581), at a 4:1 ratio of spleen cells to sp2/0 cells, in the presence of 0.3 ml of 30% polyethylene glycol, PEG 1450. After incubation at 37°C for 6 hours, the fused cells were distributed in 0.2 ml aliquots into 96-well plates at concentrations of 2 x 10⁴ SP2/0 cells per well. Feeder cells, in the form of 5 x 10⁴ normal BALB/c spleen cells, were added to each well.

The growth medium used consisted of RPMl-1640 medium, 10% heat-inactivated fetal bovine serum (FBS) (Hyclone), 0.1 mM MEM nonessential amino acids, 1 mM sodium pyruvate, 2mM L-glutamine, 100 U/ml penicillin, 100 µg/ml streptomycin (GIBCO Laboratories) and, for selection, hypoxanthine-aminopterin- thymidine (HAT) (Boehringer Mannheim).

A solid-phase radioimmunoassay (RIA) was employed for screening supernatants for the presence of mAbs specific for rhTNFα. This assay is described in Example II, below. The background binding in this assay was about 500 cpm. A supernatant was considered positive if it yielded binding of 2000 cpm or higher.

Of 322 supernatants screened, 25 were positive by RIA. Of these 25, the one with the highest binding (4800 cpm) was designated A2. Positive wells were subcloned at limiting dilution on mouse feeder cells. Upon further analysis of the supernatants in neutralization assays, A2 was found to be the only positive clone showing potent inhibiting and/or neutralizing activity. Thus, the hybridoma line A2 was selected. This line was maintained in RPMl-1640 medium with 10% FBS (GIBCO) , 0.1 mM nonessential amino acids, 1 mM sodium pyruvate, 2 mM L-glutamine, 100 U/ml penicillin and 100 µg/ml streptomycin.

Alternatively, anti-TNF antibodies which inhibit TNF biological activity can be screened by binding to peptide including at least 5 amino acids of residues 87-108 or both residues 59-80 and 87-108 of TNF (of SEQ ID NO:1) or combinations of peptides contained therein, which are used in place of the rTNF protein, as described above.

### EXAMPLE II

### Characterization of an Anti-TNF antibody of the present invention.

### A. Radioimmunoassays

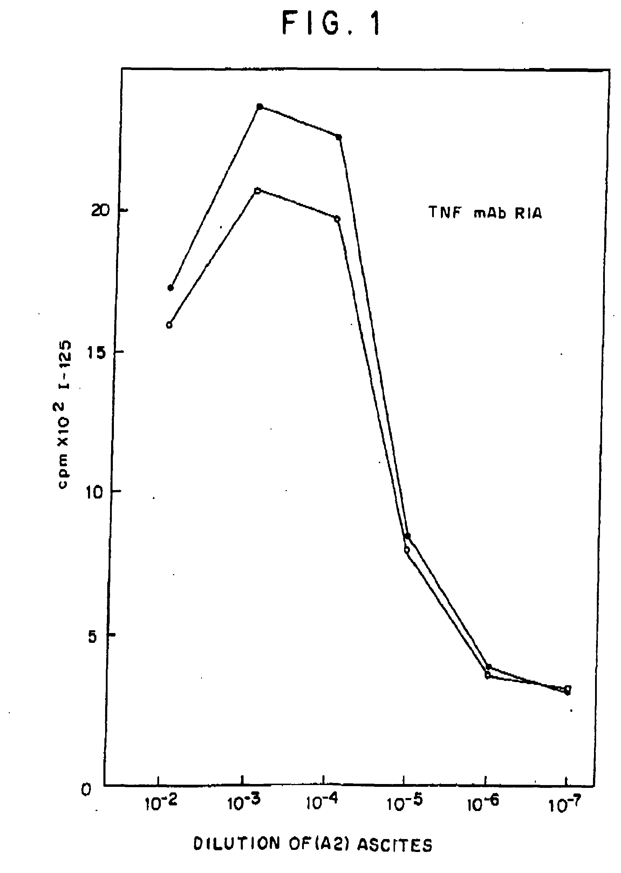

E. coli-derived rhTNF was diluted to 1 µg/ml in BCB buffer, pH 9.6, and 0.1 ml of the solution was added to each assay well. After incubation at 4°C overnight, the wells were washed briefly with BCB, then sealed with 1% bovine serum albumin (BSA) in BCB at 37°C for 1 hr. The wells were then washed 3 times with PBS containing 0.05% Tween-20 (PBS-Tween), and 70 *µ*1 diluted A2 ascites fluid was added to each well. The wells were incubated for 2 hr at 37°C, and washed 3 times with PBS-Tween. Thereafter, approximately 50,000 cpm of ¹²⁵I-labeled F(ab')2 fragment of sheep anti-mouse Ig antibodies in 50 *µ*l of PBS-Tween containing 1% BSA was added to each well, and the wells were incubated for an additional 2 hr at 37°C. The wells were washed 4 times with PBS-Tween, cut out and counted individually. Results of two determinations are shown in Figure 1.

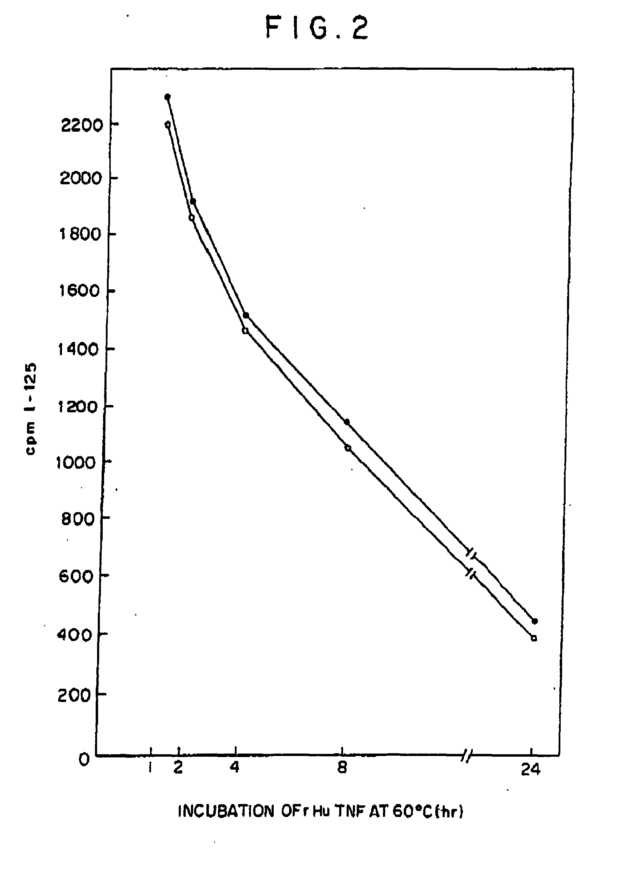

rhTNF at 5 µg/ml in PBS was heated to 60°C. At various time points, aliquots of the heat-treated TNF preparation were quickly cooled to 4°C, diluted 5-fold in BCB, and used to coat the RIA microplate wells. The RIA was carried out exactly as described above. Results from two determinations are shown in Figure 2. As incubation at 60°C substantially reduced the biological activity of hTNFα, this experiment shows mAb A2 fails to bind heat-inactivated human **TNFa.**

### B. Neutralization Assays

Samples of A2 and cA2 were purified by protein A affinity chromatography from hybridoma tissue culture supernatants of cell lines designated C134A and C168A (described above), respectively, and diafiltered in phosphate buffered saline pH 7.2 (PBS).

The toxic effect of TNF on certain tumor cell lines has been adapted as an in vitro measure of TNF levels in laboratory samples and biological fluids. The assay method of Feinman et al.,J- Immunol 138:635-640 (1987), as modified by Aderka et al., J- Immunol. 143:3517-3523 (1989), employing the TNF-sensitive target cell A673 (a human rhabdomyosarcoma cell line), was used to investigate the ability of A2 to inhibit or neutralize TNF toxicity.

Cultured human A673/6 cells were incubated with 40 pg/m1 of natural (Genzyme, Boston, MA) or recombinant (Suntory, Osaka, Japan) human TNFα with varying concentrations of mAb A2 in the presence of 20 *µ*g/ml cycloheximide at 39°C overnight. Controls included medium alone or medium + TNF in each well. Cell death was measured by staining with naphthol blue-black, and the results read spectrophotometrically at 630 nm. Absorbance at this wave length correlates with the number of live cells present.

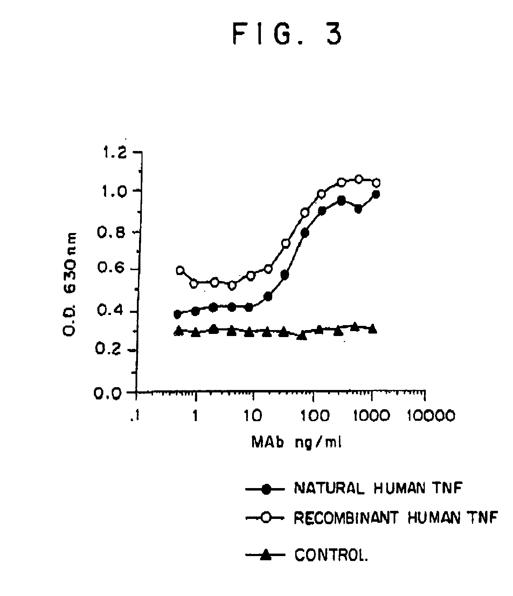

It was found that A2 inhibited or neutralized the cytotoxic effect of both natural and rhTNF in a dose-dependent manner (Figure 3).

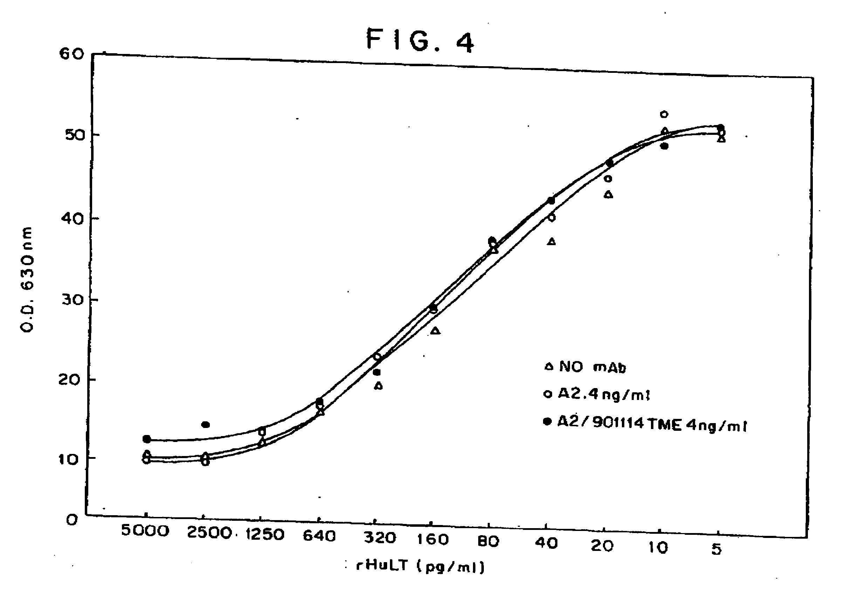

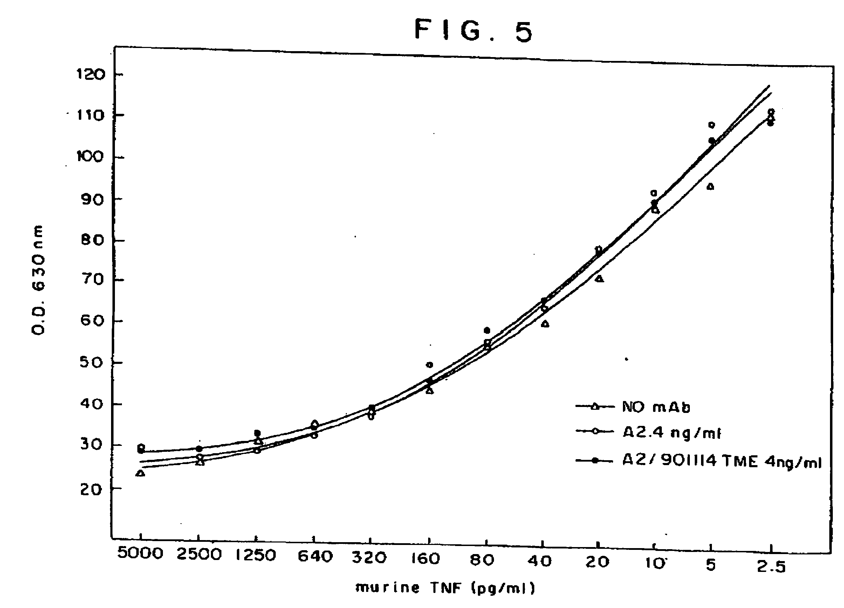

In another experiment, the specificity of this inhibiting and/or neutralizing activity was tested. A673/6 cells were seeded at 3 x 10⁴ cells/well 20 hr before the TNF bioassay. Two-fold serial dilutions of rhTNF, E. coli-derived recombinant human lymphotoxin (TNFS), and E. coli*-*derived recombinant murine TNF were prepared. The A2 hybridoma supernatant was added to an equal volume of the diluted TNF preparations, and the mixtures were incubated at room temperature for 30 min. Aliquots of 0.1 ml were transferred to the wells containing A673/6 cells, 20 µg/ml of cycloheximide was added, and the cells were incubated at 39°C overnight. The cells were then fixed and stained for evaluation of cytotoxicity. The results indicate that mAb A2 Specifically inhibited or neutralized the cytotoxicity of rhTNFα, whereas it had no effect on human lymphotoxin (TNFß) (Figure 4) or murine TNF (Figure 5).

Experiments were next performed to analyze the cross-reactivity of mAb A2 with TNF derived from non-human primates.
Monocytes isolated from B514 (baboon), J91 (cynomolgus) and RH383 (rhesus) blood by Ficoll gradient centrifugation and adherence, were incubated at 1 x 10⁵ cells/well in RPM1 1640 medium with 5% FBS and 2 µg/ml of E. coli LPS for 3 or 16 hr at 37°C to induce TNF production. Supernatants from duplicate wells were pooled and stored at 4°C for less than 20 hr until the TNF bioassay was performed, as described above, using A673/6 cells. Two-fold dilutions of the culture supernatants were mixed with either medium or purified mAb A2 at a final concentration of 1 µg/ml, incubated at room temperature for 30 min and aliquots transferred to the indicator cells. The results showed that mAb A2 failed to significantly inhibit or neutralize the cytotoxic activity of TNF produced by baboon, cynomolgus and rhesus monkey monocytes.

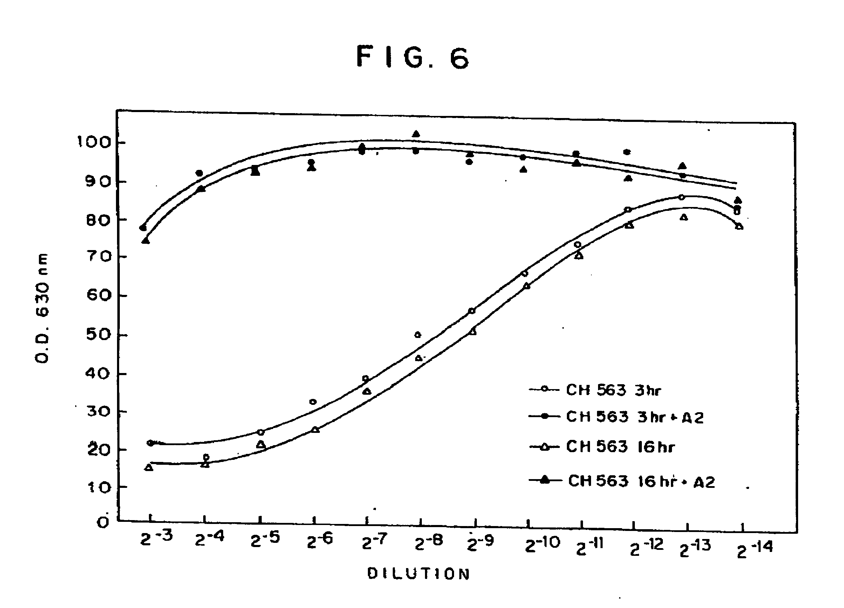

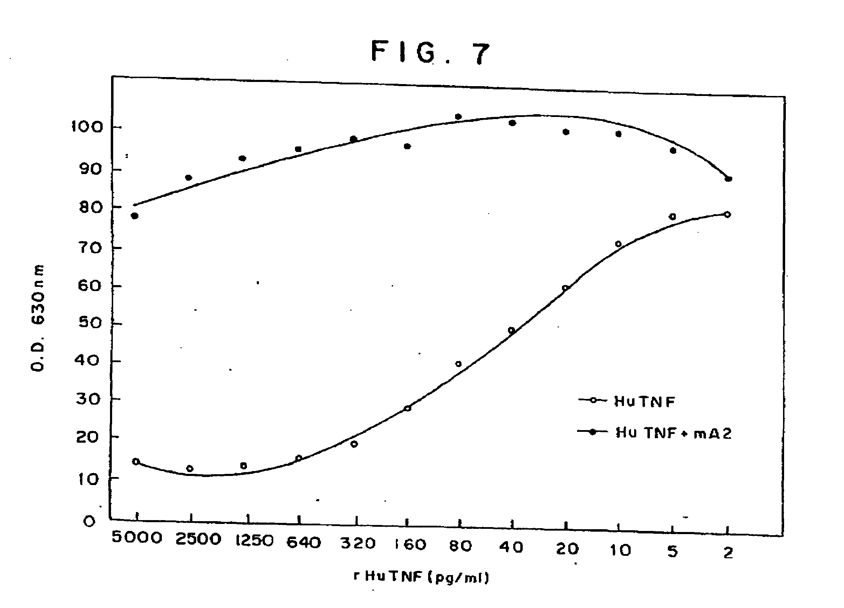

A further experiment was conducted with chimpanzee TNF. Monocytes isolated from CH563 (chimpanzee) blood were incubated as described above to generate TNF-containing supernatants. The ability of 10 µg/ml of mAb A2 to inhibit or neutralize the bioactivity of these supernatants was assayed as above. Human TNF was used as a positive control. Results, shown in Figure 6, indicate that mAb A2 had potent inhibiting and/or neutralizing activity for chimpanzee TNF, similar to that for human TNF (Figure 7).

The inhibiting and/or neutralizing activity of mAb A2 was compared with three other murine mAbs specific for human TNF, termed TNF-1, TNF-2 and TNF-3, and a control mAb. Two-fold serial dilutions of purified mAbs were mixed with rhTNF (40 pg/ml), incubated at room temperature for 30 min, and aliquots tested for TNF bioactivity as above. It was found that mAbs TNF-1, TNF-2 and TNF-3 each had a similar moderate degree of inhibiting and/or neutralizing activity. In contrast, mAb A2 had much more potent inhibiting and/or neutralizing activity.

### EXAMPLE III

### General strategy for Cloning Antibody V and C Genes

The strategy for cloning the V regions for the H and L chain genes from the hybridoma A2, which secretes the anti-TNF antibody described above, was based upon the linkage in the genome between the V region and the corresponding J (joining) region for functionally rearranged (and expressed) Ig genes. J region DNA probes can be used to screen genomic libraries to isolate DNA linked to the J regions. Although DNA in the germline configuration (i.e., unrearranged) would also hybridize to J probes, this DNA would not be linked to a Ig V region sequence and can be identified by restriction enzyme analysis of the isolated clones.

The cloning utilized herein was to isolate V regions from rearranged H and L chain genes using J_{H} and Jₖ probes. These clones were tested to see if their sequences were expressed in the A2 hybridoma by Northern analysis. Those clones that contained expressed sequence were cloned into expression vectors containing human C regions and transfected into mouse myeloma cells to determine if an antibody was produced. The antibody from producing cells was then tested for binding specificity and functionally compared to the A2 murine antibody.

### EXAMPLE IV

### Construction of a L Chain Genomic Library

To isolate the L chain V region gene from the A2 hybridoma, a size-selected genomic library was constructed using the phage lambda vector charon 27. High molecular weight DNA was isolated from A2 hybridoma cells and digested to completion with restriction endonuclease HindIII. The DNA was then fractionated on a 0.8% agarose gel and the DNA fragments of three different size ranges of approximately 3 kb, 4 kb and 6 kb were isolated from the gel by electroelution. The size ranges for library construction were chosen based upon the size of HindIII fragments that hybridized on a southern blot with the Jₖ probe. After phenol/chloroform extraction and ethanol precipitation, the DNA fragments from each size class were ligated with lambda charon 27 arms and packaged into phage particles in vitro using Gigapack Gold from Stratagene (LaJolla, CA).

These libraries were screened directly at a density of approximately 20,000 plaques per 150 mm petri dish using a ³²P-labeled Jₖ probe. The mouse L chain Jₖ probe was a 2.7 kb HindIII fragment containing all five Jₖ segments. The probe was labeled with ³²P by random priming using a kit obtained from Boehringer Mannheim. Free nucleotides were removed by centrifugation through a Sephadex G-50 column. The specific activities of the probe was approximately 10⁹ cpm/µg.

Plaque hybridizations were carried out in 5× SSC, 50% formamide, 2x Denhardt's reagent, and 200 *µ*g/ml denatured salmon sperm DNA at 42°C for 18-20 hours. Final washes were in 0.5x SSC, 0.1% SDS at 65°C. Positive clones were identified after autoradiography.

### EXAMPLES V

### Construction of H Chain Genomic Library

To isolate the v region gene for the A2 H chain, a genomic library was constructed in the lambda gtl0 vector system. High molecular weight DNA was digested to completion with restriction endonuclease ECORI and fragments of approximately 7.5 kb were isolated after agarose gel electrophoresis. These fragments were ligated with lambda gt10 arms and packaged into phage particles in vitro using Gigapack Gold.

This library was screened at a density of 20,000 plagues per 150 mm plate using a **J_{$}** probe. The Jₖ probe was a 2kb BmaHI/EcoRI fragment containing both J3 and J4 segments.
The probe was labeled as in Example III and had a similar specific radioactivity. Hybridization and wash conditions were identical to those used in Example III.

### EXAMPLE VI

### Cloning of the TNF. Specific V gene regions

Several positive clones were isolated from the H and L chain libraries after screening approximately 10° plaques from each library using the J_{H} and Jₖ probes, respectively. Following plaque purification, bacteriophage DNA was isolated for each positive clone, digested with either EcoRI (H chain clones) or HindIII (L chain clones) and fractionated on 1% agarose gels. The DNA was transferred to nitrocellulose and the blots were hybridized with the J_{H} or the J_{K} probe.

Several E chain clones were obtained that contained 7.5 kb EcoRI DNA fragments that hybridized to the J_{H} probe. For the light chain libraries, several clones from each of the three size-selected libraries were isolated that contained HindIII fragments that hybridize to the Jₖ probe. For the L chain, several independently derived HindIII fragments of 2.9 kb from the 2 kb library hybridized with a 1250 bp mRNA from A2, but not with SP2/0 mRNA (see Example VII). In addition, several HindIII fragments derived from the 4 kb library hybridized both to the A2 mRNA and the fusion partner mRNA. A 5.7 kb HindIII fragment from the 6 kb library did not hybridize to either RNA.

The observed lengths of hybridizing A2 mRNA were the correct sizes for H and L chain mRNA, respectively. Because the RNA expression was restricted to the A2 hybridoma, it was assumed that the 7.5 kb H chain fragments and the 2.9 kb L chain fragments contained the correct V region sequences from A2. One example of each type was chosen for further study. The important functional test is the demonstration that these V regions sequences, when combined with appropriate C region sequences, are capable of directing the synthesis of an antibody with a specificity and affinity similar to that of the murine A2-antibody.

The 7.5 kb H chain fragment and the 2.9 kb L chain fragment were subcloned into plasmid vectors that allow expression of the chimeric mouse/human proteins in murine myeloma cells (see Examples VIII and IX). These plasmids were co-transfected into SP2/0 cells to ascertain if intact antibody molecules were secreted, and if so, if they were of the correct specificity and affinity. Control transfections were also performed pairing the putative anti-TNF H chain with an irrelevant, but expressed, L chain; the putative anti-TNF L chain was also paired with an irrelevant, but expressed, H chain. The results indicated that the 7.5 kb H chain fragment could be expressed, whereas the 2.9 kb L chain fragment could not. This was confirmed by DNA sequence analysis that suggested portions of the coding region were not in the proper amino acid reading frame when compared to other known L chain amino acid sequences.

Because the 2.9 kb HindIII fragment appeared not to contain a functional V gene, the 4.0 kb and 5.7 kb HindIII fragments isolated from L chain libraries were cloned into expression vectors and tested for expression of chimeric antibody after co-transfection with the 7.5 kb H chain. The 5.7 kb HindIII fragment was incapable of supporting antibody expression, whereas the 4.0 kb HindIII fragment did support antibody expression. The antibody resulting from the co-transfection of the 7.5 kb putative H chain V region and the 4.0 kb L chain V region was purified, tested in solid phase TNF binding assay, and found to be inactive. It was concluded that the V region contained on the 4.0 kb HindIII fragment was not the correct anti-TNF V regions, but was contributed to the hybridoma by the fusion partner. This was subsequently confirmed by sequence analysis of cDNA derived from the A2 hybridoma and from the fusion partner.

Other independently derived L chain clones containing 2.9 kh HindIII fragments that hybridized with A2 mRNA were characterized in more detail. Although the restriction maps were similar, the clones fell into two classes with respect tot the presence or absence of an AccI enzyme site. The original (non-functional) 2.9 kb fragment (designated clone 8.3) was missing an AccI site present in some other clones (represented by clone 4.3). The DNA sequence of clone 4.3 was extremely similar to clone 8.3, but contained a single amino acid reading frame with close homology to known L chains, unlike clone 8.3. The 2.9 kb HindIII fragment from clone 4.3 was subcloned into the L chain expression vector and co-transfected with the putative anti-TNF H chain into SP2/0 cells. An antibody was synthesized, purified and tested in the solid phase TNF binding assay. This antibody bound to TNF, and therefore, the clone 4.3 L chain V region was assumed to be the correct one.

The A2 murine hybridoma has been shown to contain at least four rearranged L chain V region genes. At least two of these are expressed as proteins: clone 4.3 (the correct anti-TNF L chain gene) and the gene contained in the 4.0 kb HindIII fragment (contributed by the fusion partner). The expression of two L chains implies that the resulting antibody secreted from the murine hybridoma is actually a mixture of antibodies, some using the correct L chain, some using the incorrect L chain, and some using one of each. The presence of two different L chains in the murine A2 antibody has been confirmed by SDS gel and N-terminal protein sequence analysis of the purified antibody. Because construction of the chimeric A2 antibody involves cloning the individual H and L chain genes and expressing them in a non-producing cell line, the resulting antibody will have only the correct L chain and therefore should be a more potent antibody (see Examples X, XI and XII).

### EXAMPLE VII

### Northern Analysis of Cloned DNA

Cloned DNA corresponding to the authentic H and L chain V regions from the A2 hybridoma would be expected to hybridize to A₂ mRNA. Non-functional DNA rearrangements at either the H or L chain genetic loci should not be expressed.

Ten µg total cellular RNA was subjected to electrophoresis on 1% agarose/formaldehyde gels (Sambrook et al, supra) and transferred to nitrocellulose. Blots were hybridized with random primed DNA probes in 50%. formamide, 2x Denhardt's solution, 5x SSC, and 200 µg/ml denatured salmon sperm DNA at 42°C for 10 hours. Final wash conditions were 0.5 x SSC, 0.1% see at 65°C.

The subcloned DNA fragments were labeled with ³²P by random priming and hybridized to Northern blots containing total RNA derived from A2 cells or from cells of SP2/0, the fusion partner parent of A2. The 7.5 kb EcoRI E chain fragment hybridized with a 2 kb mRNA from A2, but not with SP2/0 mRNA. Similarly, the 2.9 kb L chain HindIII fragment (clone 4.3) hybridized with a 1250 bp mRNA from A2, but not with SP2/0 mRNA. The observed lengths of A2 mRNA hybridizing were the correct sizes for H and L chain mRNA, respectively, confirming that the v region sequences on these DNA fragments are expressed in A2 hybridoma cells.

### EXAMPLE VIII

### Construction of Expression Vectors

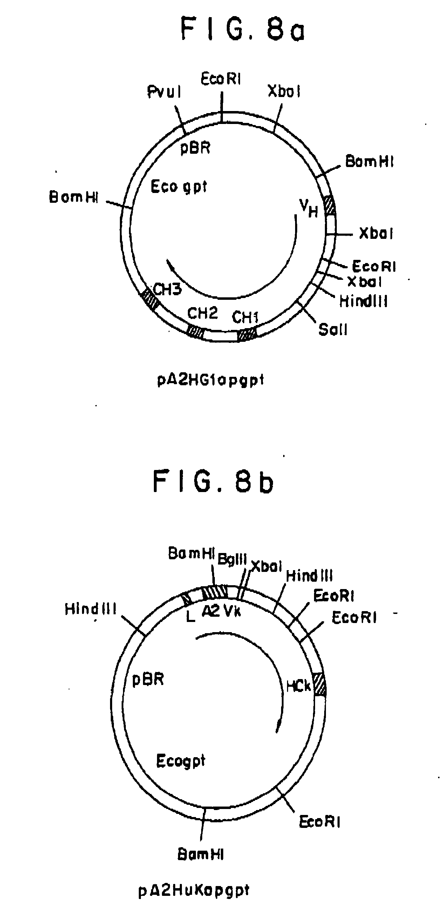

The putative L (clone 4.3) and H chain V genes described above were joined to human kappa and gamma1 constant region genes in expression vectors. The 7.5 kb EcoRI fragment corresponding to the putative V_{H} region gene from A2 was cloned into an expression vector containing the human Cₚᵤₙᵢₗ gene and the Ecogpt gene to yield the plasmid designated pA2HGlapgpt (see Figure 8).

The 2.9 kb putative V_{L} fragment from clone 4.3 was cloned into a vector containing the human kappa Cₖ gene and the Ecogpt gene to allow selection in mammalian cells. The resulting plasmid was designated pA2HuKapgpt (See Figure 8).

### EXAMPLE IX

### Expression of Chimeric Antibody Genes

To express the chimeric H and L chain genes, the expression plasmids were transfected into cells of the non-producing mouse myeloma cell line, SP2/0. Plasmid DNA to be transfected was purified by centrifuging to equilibrium in ethidium bromide/cesium chloride gradients twice. Plasmid DNA (10-50 *µ*g) was added to 10⁷ SP2/0 cells in medium containing Hank's salts, and the mixture was placed in a BioRad electroporation apparatus. Electroporation was performed at 20 volts, following which the cells were plated in 96 well microtiter plates.

Mycophenolic acid selection was applied after 24 hours and drug resistant colonies were identified after 1-2 weeks. Resistant colonies were expanded to stable cell lines and tissue culture supernatant from these cell lines was tested for antibody using an ELISA assay with goat anti-human IgG Pc antibody and goat anti-human H+L conjugated with alkaline phosphatase (obtained from Jackson Laboratories).

The chimeric A2 antibody was purified from tissue culture supernatant by Protein A- Sepharose chromatography. The supernatant was adjusted to 0.1M Tris, 0.002M EDTA, pH 8.0 and loaded on a Protein A-Sepharose column equilibrated in the same buffer. The IgG was eluted with 0.1M citrate, pH 3.5, inhibited or neutralized with 1M Tris, and dialyzed into phosphate buffered saline (PBS).

The purified chimeric antibody was evaluated for its binding and inhibiting and/or neutralizing activity.

### EXAMPLE X

### Specificity of an Anti-TNF Chimeric Antibody

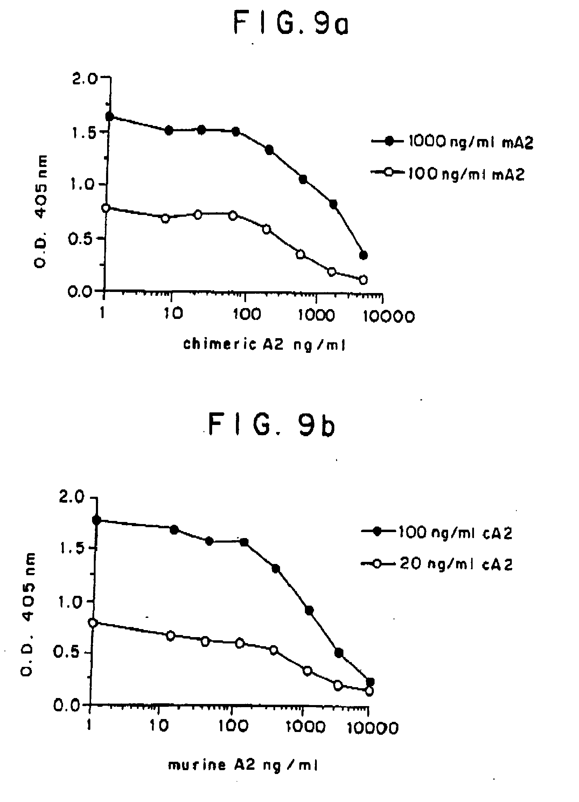

Since the antigen binding domain of cA2 was derived from murine A2, these mAbs would be expected to compete for the same binding site on TNF. Fixed concentrations of chimeric A2 and murine mAb A2 were incubated with increasing concentrations of murine and chimeric A2 competitor, respectively, in a 96-well microtiter plate coated with rhTNF (Dainippon, Osaka, Japan). Alkaline-phosphatase conjugated anti-human immunoglobulin and anti-mouse immunoglobulin second antibodies were used to detect the level of binding of chimeric and murine A2, respectively. Cross-competition for TNF antigen was observed in this solid-phase ELISA format (Figure 9). This finding is consistent with the expected identical epitope specificity of cA2 and murine A2.

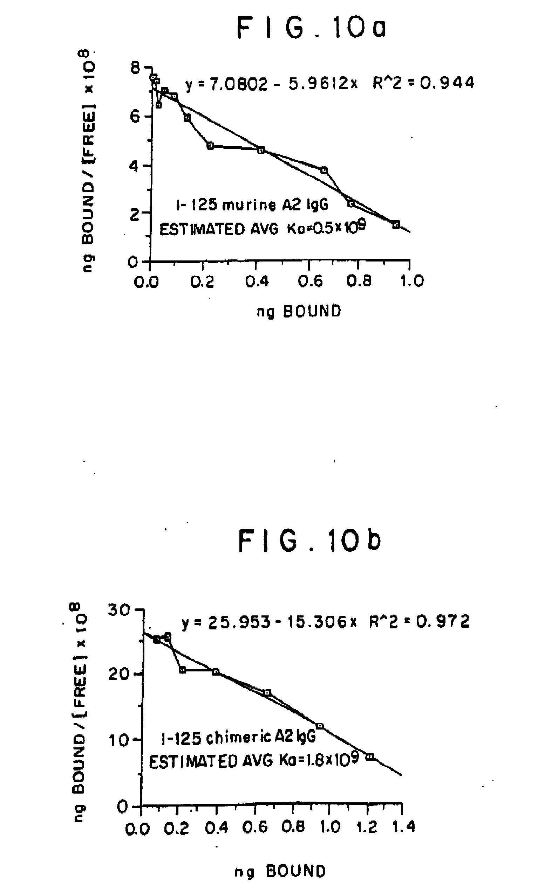

The affinity constant for binding of mouse mAb A2 and cA2 to rhTNFα was determined by Scatchard analysis (see, for example, Scatchard, G., Ann. N.Y. Acad. Sci, 51:660 (1949)). The results are shown in Figure 10. This analysis involved measuring the direct binding of ¹²⁵I labelled cA2 to immobilized rhTNFα in a 96-well plate. The antibodies were each labelled to a specific activity of about 9.7 *µ*Ci/*µ*g by the iodogen method. An affinity constant (Ka) of 0.5 x 10⁹ liters/mole was calculated for the mouse mAb A2. Unexpectedly, the chimeric A2 antibody had a higher affinity, with a Ka of 1.8 x 10⁹ liters/mole. Thus, the chimeric anti-TNFO! antibody of the present invention was shown to exhibit a significantly higher affinity of binding to human TNFα than did the parental murine A2 mAb. This finding was surprising, since murine and chimeric antibodies, fragments and regions would be expected to have affinities that are equal to or less than that of the parent mAb.

Such high affinity anti-TNF antibodies, having affinities of binding to TNFα of at least 1 x 10¹ M¹, more preferably at least 1 x 10⁹ M⁻¹ (expressed as Ka) are preferred for immunoassays which detect very low levels of TNF in biological fluids. In addition, anti-TNF antibodies having such high affinities are preferred for therapy of TNF-α-mediated conditions or pathology states.

The specificity of cA2 for TNF was confirmed by testing for cross-neutralization of human lymphotoxin (TNF-ß). Lymphotoxin shares some sequence homology and certain biological activities, for example, tumor cell cytotoxicity, with TNF (Pennica, D. et al., Nature 312:724-729 (1984)). Cultured human A673 cells were incubated with increasing concentrations of human lymphotoxin (Genentech, San Francisco, CA) with or without 4 µg/ml chimeric A2 in the presence of 20 µg/ml cycloheximide at 39°C overnight. Cell death was measured by vital staining with naphthol blue-black, as above. The results indicated that cA2 was ineffective at inhibiting and/or neutralizing human lymphotoxin, confirming the TNFα-specificity of the chimeric antibody.

The ability of A2 or cA2 to react with TNF from different animal species was also evaluated. As mentioned earlier, there are multiple epitopes on human TNF to which inhibiting and/or neutralizing mAbs will bind (Moller, A. et al., supra). Human TNF has bioactivity in a wide range of host animal species. However, certain inhibiting and/or neutralizing epitopes on human TNF are conserved amongst different animal species and others appear to be restricted to humans and chimpanzees.

Neutralization experiments utilized endotoxin-activated cell supernatants from freshly isolated human, chimpanzee, rhesus and cynomolgus monkey, baboon, pig, dog, rabbit, or rat monocytes as the TNF source. As discussed above, murine mAb A2 inhibited or neutralized activity of only human and chimpanzee TNF, and had no effect on TNF derived from other primates and lower animals. A2 also did not inhibit or neutralize the cytotoxic effect of recombinant mouse TNF.

Thus, the epitope recognized by A2 is one shared by human and chimpanzee TNFα. Chimeric A2 was also tested in this manner for cross-reactivity with monocyte-derived TNF from rat, rabbit, dog and pig, as well as with purified recombinant mouse TNFα, and natural and recombinant human TNFα. Chimeric A2 only inhibited or neutralized natural and recombinant human TNFα. Therefore, cA2 appears to share species specificity with murine A2.

### EXAMPLY XI

### In Vitro Activity and Neutralization Efficacy of a Chimeric Anti-TNF Antibody

Both the murine and chimeric anti-TNFα antibodies, A2 and cA2 were determined to have potent TNF-inhibiting and/or neutralizing activity. In the TNF cytotoxicity assay described above, murine A2, at a concentration of about 125 ng/ml completely inhibited or neutralized the biological activity of a 40 pg/m1 challenge of rhTNFα. Two separate determinations of inhibiting and/or neutralizing potency, expressed as the 50% Inhibitory Dose (ID50) were determined to be 15.9 ± 1.01 and 17.9 ± 1. 6 ng/ml (Mean ± Std error). Thus the mAb A2 has an ID50 of about 17 ng/ml.

In this same experimental system, three other marine anti-TNFα antibodies (termed TNF-1, TNF-2 and TNF-3) of comparable binding affinity to TNF were found to have ID50 values of 1-2 orders of magnitude greater, and thus were significantly less potent in neutralization than A2.

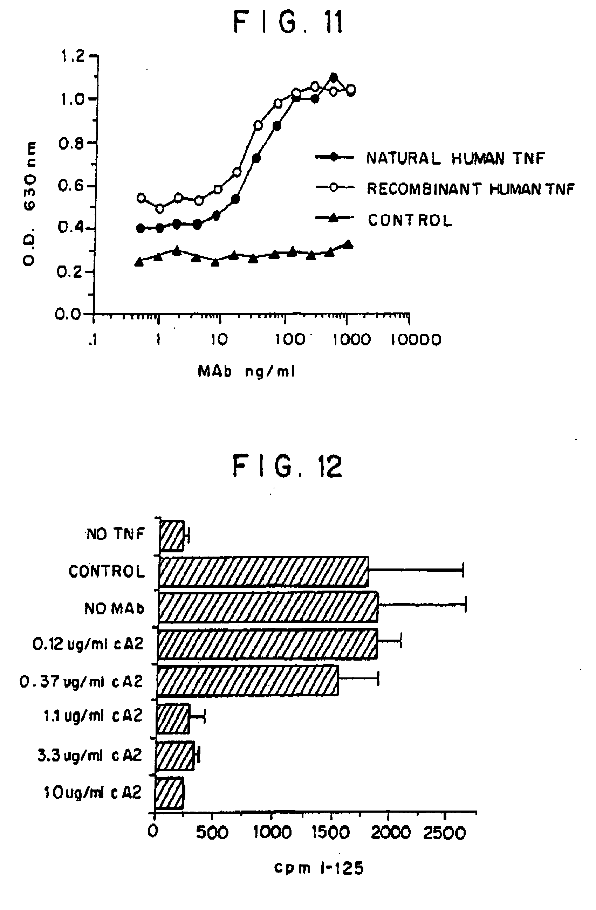

The ability of cA2 to inhibit or neutralize human THFα bioactivity in vitro was tested using the bioassay system described above. Cultured A673 cells were incubated with 40 pg/ml natural (Genzyme, Boston, MA) or recombinant (Suntory, Osaka, Japan) human TNF with or without antibody overnight as above, and cell death was measured by vital staining. As expected based upon the above results with the A2 mouse mAb, cA2 also inhibited or neutralized both natural and rhTNF in a dose-dependent manner in the cytotoxicity assay (Figure 11). In this assay format, levels of ca2 as low as 125 ng/ml completely abolished the toxic activity of TXF. Upon repeated analysis, the cA2 exhibited greater TNF-inhibiting and/or neutralizing activity than did the parent murine A2 mAb. Such inhibiting and/or neutralizing potency, at antibody levels below 1 µg/ml, can easily be attained in the blood of a subject to whom the antibody is administered. Accordingly, such highly potent inhibiting and/or neutralizing anti-TNF antibodies, in particular the chimeric antibody, are preferred for therapeutic use in TNFα-mediated pathologies or conditions.

As mentioned above, TNF induces cellular secretion of IL-6. Furthermore, there is evidence that IL-6 is involved in the pathophysiology of sepsis, although the precise role of IL-6 in that syndrome is unclear (Fong, Y. et al., J Exp Med 170:1627-1633 (1989); Starnes Jr., H.F. et al., J Immunol 145:4185-4191 (1990)). The ability of cA2 to inhibit or neutralize TNF-induced IL-6 secretion was evaluated using cultured human diploid FS-4 fibroblasts. The results in Table 1 show that cA2 was effective in blocking IL-6 secretion in cells that had been incubated overnight with TNF. TNF-induced IL-6 secretion was not inhibited in the absence of a mAb or in the presence of a control mAb specific for an irrelevant antigen.

| **TABLE 1** | | | | |
|---|---|---|---|---|
| IN VITRO NEUTRALIZATION OF TNF-INDUCED IL-6 SECRETION TNF Concentration (ng/ml) | | | | |
| Antibody | 0 | 0.3 | 1.5 | 7.5 |
| None | <0.20 | 1.36 | 2.00 | 2.56 |
| Control mAb | <0.20 | 1.60 | 1.96 | 2.16 |
| cA2 | <0.20 | <0.20 | <0.20 | 0.30 |
| Values represent mean concentrations of IL-6 of duplicate wells, in ng/ml. RhTNF (Suntory, Osaka, Japan), with or without 4 µg/ml antibody, was added to cultures of FS-4 fibroblasts and after 18 h, the supernatant was assayed for IL-6 using the Quantikine Human IL-6 Immunoassay (from R&D Systems, Minneapolis, MN). Control mAb = chimeric mouse/human IgG1 anti-platelet mAb (7E3). | | | | |

The ability of TNF to activate procoagulant and adhesion molecule activities of endothelial cells (EC) is thought to be an important component of pathology pathophysiology. In particular, this may be associated with the vascular damage, disseminated intravascular coagulation, and severe hypotension that is associated with the sepsis syndrome. Therefore, the ability of cA2 to block TNF-induced activation of cultured human umbilical vain endothelial cells (HUVBC) was evaluated- TNF stimulation of procoagulant activity was determined by exposing intact cultured HUVEC cells to TNF (with or without antibody) for 4 hours and analyzing a cell lysate in a human plasma clotting assay. The results in Table 2 show the expected upregulation by TNF of HUVEC procoagulant activity (reflected by a decreased clotting time). Chimeric antibody cA2 effectively inhibited or neutralized this TNF activity in a dose-dependent manner.

| TABLE 2 | | | | |
|---|---|---|---|---|
| IN VITRO NEUTRALIZATION OF TNF- INDUCED PROCOAGULANT ACTIVITY | | | | |
| TNF Concentration (ng/ml) | | | | |
| Antibody | µg/ml | 250 | 25 | 0 |
| None | - | 64 ±4^{*} | 63 ±1 | 133 ± 13 |
| Control Ab | 10.00 | 74 ± 6 | N.D. | 178 ± 55 |
| cA2 | 10.00 | 114 ±5 | 185 ± 61 | 141 ±18 |
| cA2 | 3.30 | 113 ±2 | 147 ±3 | N.D. |
| cA2 | 1.10 | 106 ±1 1 | 145 ± 8 | N.D. |
| A2 | 0.37 | 73 ±17 | 153 ±4 | N.D. |
| CA2 | 0.12 | 64 ± 1 | 118 ±13 | N.D. |

| | | | | |
|---|---|---|---|---|
| ^{*} Values represent mean plasma clotting time, in seconds (± S.D.). Clotting time was determined in normal human plasma after addition of the rhTNF (Dainippon, Osaka, Japan) with or without antibody- treated HUVEC lysate and Ca⁺⁺ at 37°C. N.D. = Not done. Control Ab is a chimeric mouse/human IgGl anti-CD4 antibody. | | | | |

In addition to stimulating procoagulant activity, TNF also induces surface expression of endothelial cell adhesion molecules such as ELAM-1 and ICAM-1. The ability of cA2 to inhibit or neutralize this activity of TNF was measured using an ELAM-1 specific detection radioimmunoassay. Cultured HUVEC were stimulated with 250 ng/ml rhTNF (Dainippon, Osaka, Japan) with or without antibody at 37°C overnight in a 96-well plate format. Surface expression of ELAM-1 was determined by sequential addition of a mouse anti-human ELAM-1 mAb and ¹²⁵I-labelled rabbit anti-mouse immunoglobulin second antibody directly to culture plates at 4°C.

As shown in Figure 12, TNF induced the expression of ELAM-1 on the surface of cultured HUVEC cells, and this activity was again effectively blocked in a dose-related manner by cA2.

Finally, TNF is known to stimulate mitogenic activity in cultured fibroblasts. Chimeric A2 inhibited or neutralized TNF-induced mitogenesis of human diploid FS-4 fibroblasts cultures, confirming the potent inhibiting and/or neutralizing capability of cA2 against a broad spectrum of in vitro TNF biological activities.

### EXAMPLE XII

### In Vivo Activity and Efficacy of cA2 Antibody

The highly restrictive species cross-reactivity of cA2 severely limits the ability to test the in vivo efficacy of this antibody in animals other than humans or chimpanzees. Nevertheless, evidence that the potent in vitro inhibiting and/or neutralizing capability of cA2 is manifest in vivo was desirable. Earlier animal studies showed that administration of TNF to experimental animals mimics the pathology state obtained with either Gram-negative bacterial infection or direct endotoxin administration (Tracey, K.J. et al., 1986. supra; Tracey, K.J. et al., 1987, supra; Lehmann, V. et al., supra).

An in vivo model wherein lethal doses of human TNF are administered to galactosamine-sensitized mice (Lehmann, V. et al., supra) was adapted for testing the capability of cA2 to inhibit or neutralize TNF in vivo. An i.p. challenge with 5 *µ*g (0.25 mg/kg) of rhTNF resulted in 80-90 percent mortality in untreated control animals and in animals treated i.v. 15-30 minutes later with either saline or 2 mg/kg control antibody (a chimeric IgGl derived from murine 7E3 anti-platelet mAb). In contrast, treatment with cA2 reduced mortality to 0-30 percent with 0.4 mg/kg of antibody, and to 0-10 percent with 20 mg/kgs. These results, summarized in Table 3, indicate that cA2 was capable of inhibiting and/or neutralizing the biological activity of TNF in vivo as well as in vitro.

| TABLE 3 | | |
|---|---|---|
| PREVENTION OF HUMAN TNF-INDUCED LETHALITY BY CHIMERIC A2 | | |
| | Outcome (Survivors/Total) | |
| Antibody | Experiment #1 | Experiment #2 |
| None | 1/10 | N.D. |
| Control Ab, 2 mg/kg | 2/10 | 1/10 |
| cA2 (2 mg/kg) (p=0.0001) | 9/10 (p=0.0055) | 10/10 |
| cA2 (0.4 mg/kg) (p=0.0001) | 7/10 (p-0.07) | 10/10 |

| | | |
|---|---|---|
| Female C3H/HeN mice were administered 5 µg rhTNF (Dainippon, Osaka, Japan) + 18 mg galactosamine i.p. and antibody was administered 15-30 minutes later i.v, Deaths were recorded 48 h post-challenge. Control MAb = chimeric mouse/human IgG1 anti-platelet MAb (7E3). N.D.= not done. p values refer to comparison with the control Ab. | | |

### EXAMPLE XIII:

### Determination of amino acid sequences (epitope) on human TNF-α recognized by cA2 mAb

Reagents The following reagents are readily available from commercial sources. FMOC-L-Ala-0Pfp, FMOC-L-Cys(Trt)-OPfp, FMOC-L-Asp(OtBu)-OPfp, FMOC-L-Glu(OtBu)-OPfp, FMOC-L-Phe-OPfp, FMDC-Gly-OPfp, FMOC-L-His(Boc)-0Pfp, FMOC-L-Ile-OPfp, FMOC-L-Lys(Boc)-OPfp, FMOC-L-Leu-OPfp, FMOC-L-Asn-OPfp, FMOC-L-Pro-OPfp, FMOC-L-Gln-OPfp, FMOC-L-Arg(Mtr)-OPfp, FMOC-L-Ser(tBu)-ODhbt, FMOC-L-Thr(tBu)-ODhbt, FMOC-L-Val-OPfp, FMOC-L-Trp-OPfp, FMOC-L-Try (tBu) -OPEp, and 1-hydroxybenotriazol (HOBT) were obtained from Cambridge Research Biochemicals. Piperidine and was obtained from Applied Biosystems, Inc. 1-Methyl-2-Pyrrolidinone (NMP) was obtained from EM Science; Methanol from JT Baker; Acetic Anhydride from Applied Biosystems, Inc., Trifluoroaccetic acid (TFA) from Applied Biosystems, Inc.; Diisopropylamne (DIEA), Triethylamine, Dithiothreitol (DTT) and Anisole from Aldrich and Hydrochloric Acid (HCl) from JT Baker.

Abbreviations: FMOC, 9-fluorenylmethoxycarbonyl; tBu t-butyl ether; OtB, t-butyl ester; Boc, t-butyloxycarbonyl; Mtr, 4-methoxy-2,3,6-trimethylbenzenesulfonyl; Trt, trityl; OPfp, pentafluorophenylester; ODhbt. oxo-benzotriazone ster;

A chimeric antibody of the present invention, designated cA2, was used to determine which portions of the TNF amino acid sequence were involved in inhibitory binding by the antibody by epitope mapping, whereby the amino acid sequences of TNF-α recognized by cA2 have been identified.

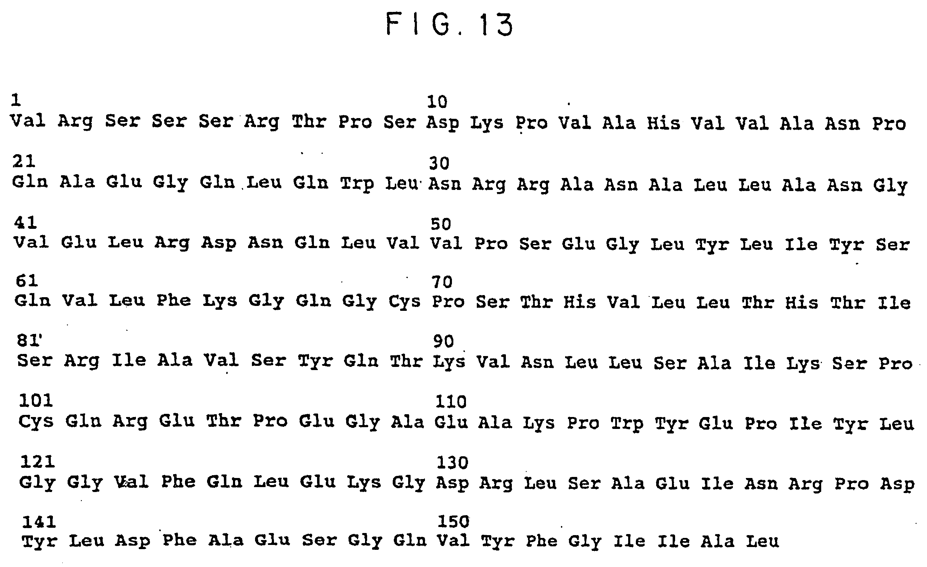

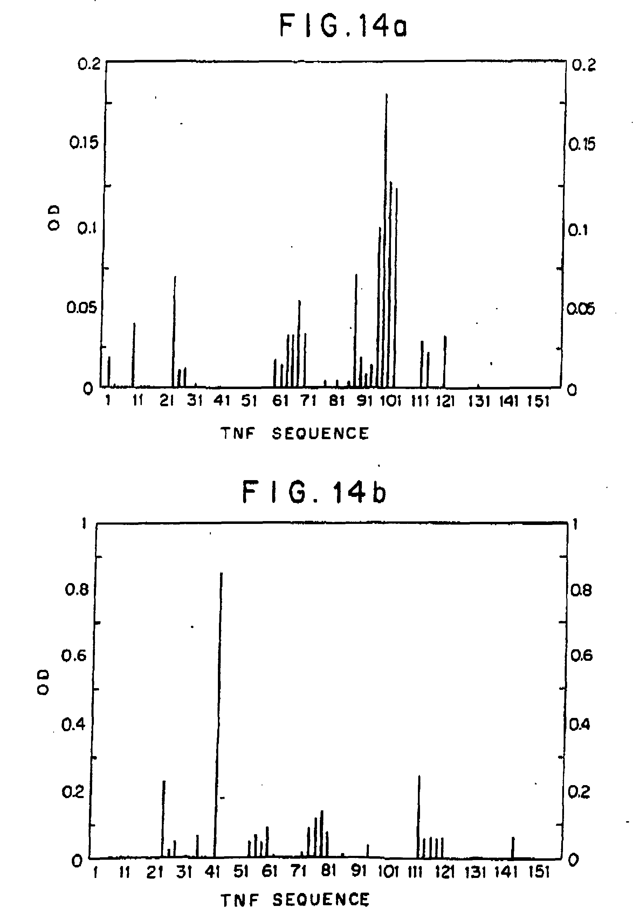

The complete primary sequence of human TNFα, according to Pennica et al, Nature 312:724-729 (1984) is shown in Figure 13. Overlapping decapeptides beginning with every second amino acid and covering the entire amino acid sequence of human TNF-α were synthesized on polyethylene pins using the method of Gysen (Gysen et al., Peptides: Chemistry and Biological, Proceedings of the Twelfth American Peptide Symposium, p. 519-523, Ed, G.R. Marshall, Escom, Leiden, 1988). Sets of peptide pins bearing free N-terminal amino groups and acetylated N-terminal amino groups were individually prepared. Both sets of peptide pins were incubated in solutions containing the anti-TNF mAb cA2 to determine the amino acid sequences that make up the cA2 epitope on human TNF-α, as described below. Figure 14A shows the results of binding to the overlapping decapeptides that comprise the entire sequence of human TNFα. The O.D. (optional density) correlates directly with the increased degree of cA2 binding. Figure 14B shows the results of binding of cA2 to the same set of peptide pins in the presence of human TNFα. This competitive binding study delineates peptides which may show non-specific binding to cA2.

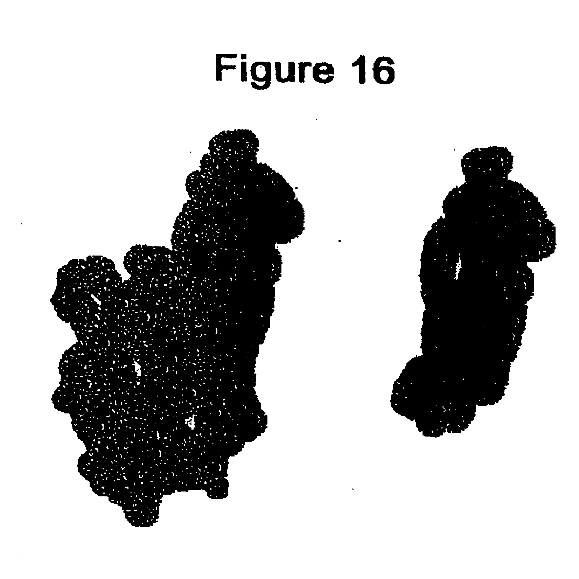

There are at least two non-contiguous peptide sequences of TNF-**α** recognized by cA2. Using the conventional protein numbering system wherein the N-terminal amino acid is number 1, the cA2 mAb recognizes an epitope composed at least in part of amino acids located within residues 87-108 or both residues 59-80 and 87-108 of TNF (of SEQ ID NO:1) . Figure 15 presents these non-contiguous sequences within the TNF sequence. These sequences are also shown in a space filling model in Figure 16B, along with a space filing model of the TNF monomer shown in Figure 16A.

Unexpectedly, the mAb cA2 blocks the action of THF-α without binding to the putative receptor binding locus, which can include one or more of, e.g., 11-13, 37-42, 49-57 or 155-157 of hTNFα (of SEQ ID NO:1). Preferred anti-TNF mAbs are those that inhibit this binding of human TNF-α to its receptors by virtue of their ability to bind to one or more of these peptide sequences. These antibodies can block the activity of TNF by virtue of binding to the cA2 epitope, such binding demonstrated to inhibit TNF activity. The identification of those peptide sequences recognized by cA2 provides the information necessary to generate additional monoclonal antibodies with binding characteristics and therapeutic utility that parallel the embodiments of this application.

Peptide Pin. Synthesis Using an epitope mapping kit purchased from Cambridge Research Biochemicals, Inc. (CRB), dodecapeptides corresponding to the entire sequence of human TNF-α were synthesized on polyethylene pins.

A synthesis schedule was generated using the CRB epitope mapping software. Prior to the first amino acid coupling, the pins were deprotected with a 20% piperidine in NMP solution for 30 minutes at room temperature. After deprotected, the pins were washed with NMP for five minutes at room temperature, followed by three methanol washes. Following the wash steps, the pins were allowed to air dry for at least 10 minutes.

The following procedure was performed for each coupling cycle:
1) The amino acid derivatives and the HOBT were weighted out according to the weights required in the synthesis schedule.
2) The HOBT was dissolved in the appropriate amount of NMP according to the synthesis schedule.
3) The amino acid derivatives were dissolved in the recommended amount of HOBT solution and 150 microliters were pipeted into the appropriate wells as directed by the well position sheet of the synthesis schedule.
4) The blocks containing the pins were placed into the wells, and the "sandwich" units stored in plastic bags in a 30°C water bath for 18 hours.
5) The pins were removed from the wells and washed once (for 5 minutes) with NMP, three times (for two minutes) with methanol and air dried for 10 minutes.
6) The pins were deprotected as described above and the procedure repeated.

To acetylate the peptides on one block of pins, the peptide pins were washed, deprotected and treated with 150 microliters of a solution containing NMP; acetic anhydride:triethylamine (5:2:1) for 90 minutes at 30°C, followed by the washing procedure outlined above. The second set of peptide pins was deprotected by not acetylated to give free N-terminal amino groups.

The final deprotection of the peptides to remove the side chain protecting groups was done using a mixture of TFA:anisole:dithiothreitol, 95:2.5:2.5 (v/v/w) for four hours at ambient temperature. After deprotection, the pins were air dried for 10 minutes, followed by a 15 minute sonication in a solution of 0.1% HCl in methanol/distilled water (1:1). The pins dried over night and were then ready for testing.

### ELISA Assay for cA2 Binding to TNF-α Peptide PINS

**Reagents :** Disruption Buffer: Sodium dihydrogen phosphate (31.2 g, Sigma cat # S-0751 or equivalent) and sodium dodecylsulfate (20.0 g, Sigma cat # L-3771 or equivalent) were dissolved in 2.0 L of milliQ water. The pH was adjusted to 7.2 ± 0.1 with 50% w/w sodium hydroxide (VWR cat # VW6730-3 or equivalent).

Blocking Buffer: Sodium dihydrogen phosphate (0.39 g, Sigma cat #S-0751 or equivalent) disodium hydrogen phosphate (1.07 g, Baker cat # 3628-1 or equivalent) and sodium chloride (8.50 g, Baker cat # 3624-5 or equivalent were dissolved in 1.0 L of milliQ water. The pH was adjusted to 7.2 ± 0.1 with 50% w/w sodium hydroxide (VWR cat VW6730-3 or equivalent). Chicken egg albumin (10.0 g, sigma cat #A-5503 or equivalent) and bovine serum albumin (10.0 g, Sigma, cat #A-3294 or equivalent) were dissolved at room temperature with gentle stirring. The solution was filtered, and to the solution was added Tween 20 (2.0 ml, Sigma cat #P-13.79 or equivalent). The solution was stirred gently at room temperature for 30 min, filtered and stored at 40°.

FBS/Tween 20: A 10 x concentrate was prepared by dissolving sodium dihydrogen phosphate (3.90 g, Sigma cat # 8-0751 or equivalent), disodium hydrogen phosphate (10.70 g, Baker cat #3828-1 or equivalent) and sodium chloride (85.0 g, Baker cat #3624-5 or equivalent) in 1.0 L of milliQ water. The pH was adjusted to 7.2 ± 0.1 with 50% w/w sodium hydroxide (VWR cat #VW 6730 or equivalent). To the solution was added Tween 20 (5.0 mL, Sigma cat #P-1379 or equivalent), and the mixture stirred gently. Just prior to use 100 mL of this solution was diluted to 1.0 L with milliQ water.

Substrate solution: Substrate buffer was prepared by dissolving citric acid (4.20g, Malinckrodt cat #0627 or equivalent) and disodium hydrogen phosphate (7.10 g, Baker cat #3828-1 or equivalent) in 1.0 L of milliQ water. The pH was adjusted to 5.00 with 50% w/w sodium hydroxide (VWR cat #VW6730-3 or equivalent). Immediately prior to use an OPD substrate tablet (30 mg, Sigma cat #P-8412 or equivalent and 30% v/v hydrogen peroxide (40 µL, Sigma cat #P-1379 or equivalent) were added to the substrate buffer 25.0 mL). The solution was wrapped in foil and mixed thoroughly.

4 NH₂SO₄; Sulfuric acid (53 mL, EM Science cat #SX1244-5 or equivalent) was slowly added to miliQ water (447 mL) and cooled to room temperature prior to use.

Equipment: Molecular Devices Model nu-max plate reader or equivalent. Scientific Products Model R4140 Oscillating table shaker and equivalent. Branson Model 5200 ultra-sonic bath or equivalent. Finnpipette Model 4172317 multichannel pipeter or equivalent. Corning Model 25801 96 well disposable polystyrene Elisa Plates.

Prior to use and after each subsequent use the peptide pins were cleaned using the following procedure. Disruption buffer (2.0 L) was heated to 60° and placed in an ultra-sonic bath in a fume hood. To the disruption buffer was added dithiolthreitol (2.5 g, Sigma cat #D-0632 or equivalent). The peptide pins were sonicated in this medium for 30 min, washed thoroughly with milliQ waster, suspended in a boiling ethanol bath for 2 min, and air-dried.

Blocking buffer (200 µL) was added to a 96 well disposable polystyrene Elisa plate and the peptide pins suspended in the wells. The peptide pins and plate were incubated for 2 h at room temperature on an oscillating table shaker. The plates and peptide pins were washed with PBS/Tween 20 (four times). To each well was added a 20 µg/ml concentration of cA2 antibody (diluted with blocking buffer, 175 µL/well). TNF competition was done by incubation of TNFα (40 µg/ml) and cA2 (20 µg/ml) in BSA/ovalbumin/BBS for three hours at room temperature. The peptide pins were suspended in the plate and incubated at 4° overnight. The peptide pins and plate were washed with PBS/Tween 20 (four times). To each well was added anti-human goat antibody conjugated to horseradish peroxidase (diluted with blocking buffer to 1/2000, 175 µL/well, Jackson ImmunoResearch Labs). The peptide pins were suspended in the plate, and incubated for 1 h at room temperature on a oscillating cable shaker. The plates and peptide pins were washed with PBS/Tween 20 (four times). To each well added freshly prepared substrate solution (150 *µ*L/well), the peptide pins were suspended in the plate and incubated for 1 h at room temperature on an oscillating table shaker. The peptide pins were removed and to each well is added 4N H₂SO₄ (50 *µ*L). The plates were read in a Molecular Devices plate reader (490 nm, subtracting 650 nm as a blank), and the results are shown in Figures 14A and 14B, as described above.

### EXAMPLE XIV

### Production Mouse Anti-Human TNF mAb Using TNF Peptide Fragments

Female BALB/c mice, as in Example I above, are injected subcutaneously and intraperitoneally (i.p-) with forty µg of purified E. coli-derived recombinant human TNF (rhTNF) fragments comprising anti-TNF epitopes of at least 5 amino acids located within the non-contiguous sequence 59-80, 87-108 or both residues 59-80 and 87-108 of TNF (of SEQ ID N0:1), as presented above, emulsified with an equal volume of complete Freund's adjuvant (obtained from Difco laboratories) in 0.4 ml is into a mouse. One week later, a booster injection of 5 µg of these rhTNF fragments in incomplete Freund's adjuvant is given i.p. followed by four consecutive i.p. injections of 10 *µ*g of TNF fragments including anti-TNF epitopes including amino acids from residues 59-80, 87-108 or both 59-80 and 87-108 of hTNFα (of SEQ ID N0:1) without adjuvant. Bight weeks after the last injection, the mouse is boosted i.p. with 10 µg of TNF.

Four days later, the mouse is sacrificed, the spleen is obtained and a spleen cell suspension is prepared. Spleen cells are fused with cells of the nonsecreting hybridoma, Sp2/0 (ATCC CRL1581), at a 4:1 ratio of spleen cells to Sp2/0 cells, in the presence of 0.3 ml of 30% polyethylene glycol, PEG 1450. After incubation at 37°C for 6 hours, the fused cells are distributed in 0.2 ml aliquots into 96-well plates at concentrations of 2 x 10⁴ SP2/0 cells per well. Feeder cells, in the form of 5 x 10⁴ normal BALB/c spleen cells, are added to each well.

The growth medium used consisted of RPM1-1640 medium, 10% heat-inactivated fetal bovine serum (FBS) (Hyclone), 0.1 mM MEM nonessential amino acids, 1 mM sodium pyruvate, 2mM L-glutamine, 100 U/ml penicillin, 100 µg/ml streptomycin (GIBCO Laboratories) and, for selection, hypoxanthine-aminopterin-thymidine (HAT) (Boehringer Mannheim).

A solid-phase radioimmunoassay (RIA) is employed for screening supernatants for the presence of mAbs specific for rhTNFα fragments including portions of residues 59-80, 87-108 or both 59-80 and 87-108 of hTNFα (of SEQ ID N0:1). This assay is described in Example II, above. The background binding in this assay is about 500 cpm. A supernatant is considered positive if it yielded binding of 2000 cpm or higher.

Of the supernatants screened, one or more positive supernatants are routinely identified by RIA. Of these positive supernatants, the highest binding (as shown by the higher cpm values) are subcloned at limiting dilution on mouse feeder cells. Upon further analysis of the supernatants in neutralization assays, routinely one or more antibodies are found to have potent inhibiting and/or neutralizing activity. These positive and inhibiting and/or neutralizing hybridoma lines are then selected and maintained in RPM1-1640 medium with 10% FBS (GIBCO), 0.1 mM nonessential amino acids, 1 mM sodium pyruvate, 2 mM L-glutamine, 100 U/ml penicillin and 100 *µ*g/ml. streptomycin.

### EXAMPLE XV

### Production of Murine and chimeric antibiodies fragments and regions from TNF peptides

Marine and chimeric antibodies, fragments and regions are obtained by construction of chimeric expression vectors encoding the mouse variable region of antibodies obtained in Example XIV and human constant regions, as presented in Examples IV-IX above.

The resulting chimeric A2 antibody is purified from tissue culture supernatant by protein A-Sepharose chromatography. The supernatant is adjusted to 0.1M Tris, 0.002M EDTA, pH 8.0 and loaded on a Protein A-Sepharose column equilibrated in the same buffer, The IgG is then eluted with 0.1M citrate, pH 3.5, neutralized with 1M Tris, and dialyzed into phosphate buffered saline (PBS).

The purified murine and chimeric antibodies, fragments and regions are evaluated for its binding and inhibiting and/or neutralizing activity.

### EXAMPLE XVI

### In Vitro Activity and Neutralization Efficacy of a Chimeric Anti-TNF Antibody

Both the murine and chimeric anti-TNFα antibodies of the present invention, as obtained according to Examples XIV and XV, are determined to have potent TNF-inhibiting and/or neutralizing activity, as shown for example, in the TNF cytotoxicity assay described above, expressed as the 50% Inhibitory Dose (ID50).

In this same experimental system, three other murine anti-TNFα antibodies (termed TNF-1, TNF-2 and TNF-3) of comparable binding affinity to TNF are found to have ID50 values of 1-2 orders of magnitude greater, and thus have significantly less potent in neutralization, than both the murine and chimeric anti-TNFα antibodies of the present invention.

The ability of both the murine and chimeric anti-TNFα antibodies of the present invention, as obtained according to Examples XIV and XV, to inhibit or neutralize human TNFα bioactivity in vitro is tested using the bioassay system described above. Cultured cells producing the murine or chimeric anti-TNFα antibodies of the present invention, as obtained according to Examples XIV and XV, are incubated with 40 pg/ml natural (Genzyme, Boston, MA) or recombinant (Suntory, Osaka, Japan) human TNF with or without antibody overnight as above, and cell death is measured by vital staining. As expected, both the murine and chimeric anti-TNFα antibodies of the present invention, as obtained according to Examples XIV and XV, inhibited or neutralized both natural and rhTNF in a dose-dependent manner in the cytotoxicity assay. Such inhibiting and/or neutralizing potency, at antibody levels below 1 µg/ml, can easily be attained in the blood of a subject to whom the antibody is administered. Accordingly, such highly potent inhibiting and/or neutralizing anti-TNF antibodies, in particular the chimeric antibody, are preferred for therapeutic use in TNFα-mediated pathologies or conditions.
The ability of cA2 to inhibit or neutralize TNF-induced IL-6 secretion is evaluated using cultured human diploid FS-4 fibroblasts. The results are expected to show that both murine and chimeric anti-TNFα antibodies of the present invention, as obtained according to Examples XIV and XV, are effective in blocking IL-6 secretion in cells that had been incubated overnight with TNF. TNF-induced IL-6 secretion is not inhibited in the absence of a mAb or in the presence of a control mAb specific for an irrelevant antigen.

The ability of TNF to activate procoagulant and adhesion molecule activities of endothelial cells (EC) is thought to be an important component of pathology pathophysiology. In particular, this may be associated with the vascular damage, disseminated intravascular coagulation, and severe hypotension that is associated with the sepsis syndrome. Therefore, the ability of both the murine and chimeric anti-TNFα antibodies of the present invention, as obtained according to Examples XIV and XV, to block TNF-induced activation of cultured human umbilical vein endothelial cells (HUVEC) is evaluated. TNF stimulation of procoagulant activity is determined by exposing intact cultured HUVEC cells to TNF (with or without antibody) for 4 hours and analyzing a cell lysate in a human plasma clotting assay. The results are expected to show the expected upregulation by TNF of HUVEC procoagulant activity (reflected by a decreased clotting time) - Both the murine and chimeric anti-TNFα antibodies of the present invention, as obtained according to Examples XIV and XV, are expected to effectively inhibit or neutralize this TNF activity in a dose-dependent manner.

In addition to stimulating procoagulant activity, TNF also induces surface expression of endothelial cell adhesion molecules such as ELAM-1 and ICAM-1. The ability of both the murine and chimeric anti-TNFα antibodies of the present invention, as obtained according to Examples XIV and XV, are expected to inhibit or neutralize this activity of TNF is measured using an ELAM-1 specific detection radioimmunoassay. Cultured HUVEC are stimulated with 250 ng/ml rhTNF (Dainippon, osaka, Japan) with or without antibody at 37°C overnight in a 96-well plate format. Surface expression of ELAM-1 is determined by sequential addition of a mouse anti-human ELAM-1 mAb and ¹²⁵I-labelled rabbit anti-mouse immunoglobulin second antibody directly to culture plates at 4°C.

TNF is expected to induce the expression of ELAM-1 on the surface of cultured HUVEC cells, and this activity is again expected to be effectively blocked in a dose-related manner by both the murine and chimeric anti-TNFα antibodies of the present invention, as obtained according to Examples XIV and XV.

Finally, TNF is known to stimulate mitogenic activity in cultured fibroblasts. Both the murine and chimeric anti-TNFα antibodies of the present invention, as obtained according to Examples XIV and XV, are expected to inhibit or neutralize TNF-induced mitogenesis of human diploid FS-4 fibroblasts cultures, confirming the potent inhibiting and/or neutralizing capability of both the murine and chimeric anti-TNFα antibodies of the present invention, as obtained according to Examples XIV and XV against a broad spectrum of *in vitro* TNF biological activities.

### EXAMPLE XVII

### In Vivo Activity and Efficacy of cA2 Antibody

An in vivo model wherein lethal doses of human TNF are administered to galactosamine-sensitized mice (Lehmann, V. et al., *supra)* is adapted for testing the capability of both the murine and chimeric anti-TNFα antibodies of the present invention, as obtained according to Examples XIV and XV, to inhibit or neutralize TNF in vivo. An i.p. challenge with 5 µg (0.25 mg/kg) of rhTNF resulted in 80-90 percent mortality in untreated control animals and in animals treated i.v. 15-30 minutes later with either saline or 2 mg/kg control antibody (a chimeric IgG1 derived from murine 7E3 anti-platelet mAb). In contrast, treatment with both the murine and chimeric anti-TNFα antibodies of the present invention, as obtained according to Examples XIV and XV, is expected to reduce mortality to 0-30 percent with 0.4 mg/kg of antibody, and to 0-10 percent with 20 mg/kgs. These expected results indicate that both the murine and chimeric anti-TNFα antibodies of the present invention, as obtained according to Examples XIV and XV, are capable of inhibiting and/or neutralizing the biological activity of TNF in vivo as well as in vitro.

### EXAMPLE XVIII

### Clinical Activity and Efficacy of cA2 Antibody

Chimeric IgG1 anti-human TNF monoclonal antibody cA2 was administered to healthy male human volunteers as patients. One hour after receiving 4 ng/kg of an NIH reference endotoxin, the volunteers were administered either saline, as a control, or 0.01, 0.10 or 10 mg/kg of cA2 in a pharmaceutically acceptable form. TNF levels in serum were measured over time and were found to show a dose dependent decrease in peak TNF levels with no TNF being detected in volunteers receiving a 10 mg/kg dose of cA2. Accordingly, therapy with an anti-TNF antibody of the present invention is expected to inhibit TNF-mediated effects in humans.

Patients receiving endotoxin develop pronounced leukopenia thought to be due to margination of white blood cells. As the white blood cells become activated, they can attach to endothelial receptors with resultant endothelial damage. At doses of 1.0 to 10.0 mg/kg, this leukopenia is prevented, whereas, at 0.01 and 0.1 mg/kg dosages, a drop in white cell count was observed. The drop was most pronounced among the polymorph cell line. In all patients there was a subsequent leukocytosis, which was unchanged by treatment with anti-TNF anti-body cA2. This blocking effect on white blood cell margination is expected to represent a protective effect against the endothelial damage associated with TNF. It is expected in the art that this TNF-related endothelial damage plays a significant role in the morbidity and mortality associated with sepsis, and it is therefore expected that the anti-TNF antibodies of the present invention will provide a protective effect against these damaging effects, as presented herein.

The references cited above are all incorporated by reference herein, whether specifically incorporated or not.

Having now fully described this invention, it will be appreciated by those skilled in the art that the same can be performed within a wide range of equivalent parameters, concentrations, and conditions without departing from the spirit and scope of the invention and without undue experimentation.

While this invention has been described in connection with specific embodiments thereof, it will be understood that it is capable of further modifications. This application is intended to cover any variations, uses, or adaptations of the inventions following, in general, the principles of the invention and including such departures from the present disclosure as come within known or customary practice within the art to which the invention pertains and as may be applied to the essential features hereinbefore set forth as follows in the scope of the appended claims. It is to be understood that the phraseology or terminology employed herein is for the purpose of description and not of limitation.

SEQUENCE LISTING

(1) GENERAL INFORMATION:

(i) APPLICANT: Le, Junming
Vilcek, Jan
Daddona, Peter E.
Ghrayeb, John
Knight, David M.
Siegel, Scott A.

(ii) TITLE OF INVENTION: MONOCLONAL AND CHIMERIC
ANTIBODIES
SPECIFIC FOR HUMAN TUMOR NECROSIS FACTOR

(iii) NUMBER OF SEQUENCES: 1

(iv) CORRESPONDENCE ADDRESS:
(A) ADDRESSEE: Browdy and Neimark
(B) STREET: 419 Seventh Street, N.W.
(C) CITY: Washington
(D) STATE: D.C.
(E) COUNTRY: USA
(F) ZIP: 20004

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0,
Version #1.25

(vi) CURRENT APPLICATION DATA:
(A) APPLICATION NUMBER:
(B) FILING DATE:
(C) CLASSIFICATION:

(vii) PRIOR APPLICATION DATA:
(A) APPLICATION NUMBER: US 07/670,827
(B) FILING DATE: 18-MAR-1991

(ix) TELECOMMUNICATION INFORMATION:
(A) TELEPHONE: 202-628-5197
(B) TELEFAX: 202-737-3528

(2) INFORMATION FOR SEQ ID NO:1:

(i) SEQUENCE CHARACTERISTICS:
(A) LENGTH: 157 amino acids
(B) TYPE: amino acid
(D) TOPOLOGY: linear

(ii) MOLECULE TYPE: peptide

(xi) SEQUENCE DESCRIPTION: SEQ ID NO:1:

## Claims

1. An immunoglobulin molecule, wherein said immunoglobulin molecule is selected from an antibody, an immunoglobulin chain, or a fragment or region thereof, said immunoglobulin molecule comprising a human immunoglobulin constant region and an immunoglobulin variable region having specificity for a neutralizing epitope of human tumor necrosis factor-alpha (TNFalpha).

2. An immunoglobulin molecule according to claim 1, comprising two light chains and two heavy chains, each of said chains comprising at least part of a constant region and at least part of a variable region, said variable region having specificity to human TNFalpha said immunoglobulin molecule binding with high affinity to a neutralizing epitope of human TNFalpha in vivo.

3. An immunoglobulin molecule according to claim 1 or claim 2, wherein: (a) binding of said immunoglobulin molecule to TNFalpha inhibits a pathologic activity of TNFalpha; (b) said immunoglobulin molecule has an antigen binding region which binds residues 87-108, or both 59-80 and 87-108, of hTNFalpha of SEQ ID NO: 1.

4. An immunoglobulin molecule according to claim 1, wherein said immunoglobulin chain is a heavy chain or a light chain.

5. An immunoglobulin molecule according to claim 2, wherein: (a) said affinity, measured as an association constant (Ka), is at least 1 x 10⁸ liter/mole, and/or (b) said immunoglobulin molecule neutralises human TNFalpha with an ID50 of at least about 1µg/ml.

6. An immunoglobulin molecule according to any of claims 1 to 5, wherein said immunoglobulin molecule (a) competitively inhibits the binding of antibody A2 to TNF and (b) binds to a neutralizing epitope of human TNFalpha.

7. An immunoglobulin molecule according to claim 6, wherein: (a) said molecule has an antigen binding region which binds residues 87-108, or both 59-80 and 87-108, of hTNFalpha of SEQ ID NO: 1.

8. An isolated TNF polynucleotide, comprising a nucleotide sequence encoding an immunoglobulin molecule according to any one of claims 1 to 7, wherein said nucleotide sequence encodes at least one variable region in operable linkage with at least one constant region.

9. A polynucleotide according to claim 8, wherein: (a) said nucleotide sequence is selected from a genomic DNA sequence or a cDNA sequence; and/or (b) said polynucleotide is an expression vehicle.

10. A host transformed or transfected with the polynucleotide according to claim 6 or claim 9, the host being an eukaryotic cell, a mammalian cell, or a bacterial cell.

11. A process for preparing an immunoglobulin molecule according to any of claims 1 to 7, comprising the steps of: (a) culturing a host according to claim 10 such that said immunoglobulin molecule is expressed in recoverable amounts; and (b) recovering said immunoglobulin molecule from said host or culture.

12. A pharmaceutical composition, comprising an immunoglobulin molecule according to any one of claims 1 to 7, and a pharmaceutically acceptable carrier.

13. The pharmaceutical composition according to claim 12 for use in a method for treating a subject having a pathology mediated by TNFalpha; wherein said pathology is selected from sepsis syndrome, cachexia, circulatory collapse and shock resulting from acute or chronic bacterial infection, a bacterial infection, a viral infection, a fungal infection, systemic lupus erythematosus, rheumatoid arthritis, alcohol-induced hepatitis, a chronic inflammatory pathology, a vascular inflammatory pathology, a graft-versus-host pathology, Kaisaki's pathology and a malignant pathology.

14. The pharmaceutical composition according to claim 13, wherein said pathology is selected from rheumatoid arthritis, a chronic inflammatory pathology, a vascular inflammatory pathology, a graft-versus-host pathology, and a malignant pathology.
